# EUROPEAN PATENT SPECIFICATION

(11) **EP 3 421 500 B1**
(45) Date of publication and mention of the grant of the patent: **05.03.2025**
(21) Application number: 16891288.9
(22) Date of filing: 16.12.2016
(51) Int. Cl.: C07K 16/46, C12N 15/62, A61K 47/68

(54) **METHOD FOR EXPRESSING AND PREPARING AN ANTIBODY FUSION PROTEIN**
VERFAHREN ZUR EXPRESSION UND HERSTELLUNG EINES ANTIKÖRPERFUSIONSPROTEINS
PROCÉDÉ D'EXPRESSION ET DE PRÉPARATION D'UNE PROTÉINE DE FUSION D'ANTICORPS

(30) Priority: 23.02.2016 CN 201610099680
(43) Date of publication of application: 02.01.2019
(73) Proprietor: Jecho Laboratories, Inc., Frederick, Maryland 21704 (US); Jecho Biopharmaceuticals Co., Ltd., Tianjin 300467 (CN)
(72) Inventor: ZHU, Jianwei, Shanghai 200240 (CN); HAN, Lei, Shanghai 200240 (CN); CHEN, Junsheng, Shanghai 200240 (CN); DING, Kai, Shanghai 200240 (CN); XIE, Yueqing, Shanghai 200240 (CN); JIANG, Hua, Shanghai 200240 (CN); LU, Huili, Shanghai 200240 (CN); ZHANG, Baohong, Shanghai 200240 (CN); ZHANG, Lei, Shanghai 200240 (CN)
(74) Representative: Weickmann & Weickmann PartmbB
(86) International application number: PCT/CN2016/110293
(87) International publication number: WO 2017/143840

(56) References cited:
- WO-A2-02/46208
- WO-A2-2004/101739
- CN-A- 106 397 598
- US-A1- 2003 157 091
- US-A1- 2008 254 512
- YAN ZHOU ET AL: "Fully human HER2/cluster of differentiation 3 bispecific antibody triggers potent and specific cytotoxicity of T lymphocytes against breast cancer", MOLECULAR MEDICINE REPORTS, 5 March 2015 (2015-03-05), GR, XP055319104, ISSN: 1791-2997, DOI: 10.3892/mmr.2015.3441
- JENS R. SYDOR ET AL: "Establishment of Intein-Mediated Protein Ligation under Denaturing Conditions: C-Terminal Labeling of a Single-Chain Antibody for Biochip Screening", BIOCONJUGATE CHEMISTRY, vol. 13, no. 4, 1 July 2002 (2002-07-01), US, pages 707 - 712, XP055549166, ISSN: 1043-1802, DOI: 10.1021/bc025534z
- ABHIJIT BHAT ET AL: "Lead discovery and optimization strategies for peptide macrocycles", EUROPEAN JOURNAL OF MEDICINAL CHEMISTRY, 1 July 2014 (2014-07-01), XP055174944, ISSN: 0223-5234, DOI: 10.1016/j.ejmech.2014.07.083
- KLINE TONI ET AL: "Methods to Make Homogenous Antibody Drug Conjugates", PHARMACEUTICAL RESEARCH, SPRINGER NEW YORK LLC, US, vol. 32, no. 11, 16 December 2014 (2014-12-16), pages 3480 - 3493, XP035553870, ISSN: 0724-8741, [retrieved on 20141216], DOI: 10.1007/S11095-014-1596-8
- CARRIE J. MARSHALL ET AL: "Facile Chemical Functionalization of Proteins through Intein-Linked Yeast Display", BIOCONJUGATE CHEMISTRY, vol. 24, no. 9, 18 September 2013 (2013-09-18), US, pages 1634 - 1644, XP055306639, ISSN: 1043-1802, DOI: 10.1021/bc4002618
- MIQUEL VILA-PERELL? ET AL: "Streamlined Expressed Protein Ligation Using Split Inteins", JOURNAL OF THE AMERICAN CHEMICAL SOCIETY, vol. 135, no. 1, 9 January 2013 (2013-01-09), pages 286 - 292, XP055240219, ISSN: 0002-7863, DOI: 10.1021/ja309126m
- SINA MÖHLMANN ET AL: "Site-specific modification of ED-B-targeting antibody using intein-fusion technology", BMC BIOTECHNOLOGY, BIOMED CENTRAL LTD. LONDON, GB, vol. 11, no. 1, 21 July 2011 (2011-07-21), pages 76, XP021105286, ISSN: 1472-6750, DOI: 10.1186/1472-6750-11-76
- GERRIT VOLKMANN ET AL: "Controllable protein cleavages through intein fragment complementation", PROTEIN SCIENCE, vol. 18, no. 11, 1 November 2009 (2009-11-01), US, pages 2393 - 2402, XP055551199, ISSN: 0961-8368, DOI: 10.1002/pro.249
- YUKI SHIBUYA ET AL: "Generation of camelid VHH bispecific constructs via in-cell intein-mediated protein trans-splicing", PROTEIN ENGINEERING, DESIGN AND SELECTION, vol. 30, no. 1, 23 November 2016 (2016-11-23), GB, pages 15 - 21, XP055548420, ISSN: 1741-0126, DOI: 10.1093/protein/gzw057
- LEI HAN ET AL: "Efficient generation of bispecific IgG antibodies by split intein mediated protein trans-splicing system", SCIENTIFIC REPORTS, vol. 7, no. 1, 21 August 2017 (2017-08-21), XP055548370, DOI: 10.1038/s41598-017-08641-3
- BENJAMIN J. UMLAUF ET AL: "Site-Specific Antibody Functionalization Using Tetrazine-Styrene Cycloaddition", BIOCONJUGATE CHEMISTRY, vol. 29, no. 5, 16 May 2018 (2018-05-16), US, pages 1605 - 1613, XP055551528, ISSN: 1043-1802, DOI: 10.1021/acs.bioconjchem.8b00114
- LEI HAN ET AL: "Naturally split intein Npu DnaE mediated rapid generation of bispecific IgG antibodies", METHODS, vol. 154, 9 October 2018 (2018-10-09), NL, pages 32 - 37, XP055548468, ISSN: 1046-2023, DOI: 10.1016/j.ymeth.2018.10.001
- LEWIS, S. M. ET AL.: "Generation of bispecific IgG antibodies by structure-based design of an orthogonal Fab interface", NATURE BIOTECHNOLOGY, vol. 32, no. 2, 26 January 2014 (2014-01-26), pages 191 - 198, XP 055240003
- SPIESS, C. ET AL.: "Bispecific antibodies with natural architecture produced by co-culture of bacteria expressing two distinct half-antibodies", NATURE BIOTECHNOLOGY, vol. 31, no. 8, 7 July 2013 (2013-07-07), pages 753 - 758, XP 055127867
- RIDGWAY, J. B. B. ET AL.: "Knobs-into-holes' engineering of antibody CH 3 domains for heavy chain heterodimerization", PROTEIN ENGINEERING, vol. 9, no. 7, 31 December 1996 (1996-12-31), pages 617 - 621, XP 002610995

## Description

### Technical Field

The present disclosure relates to the field of biological technologies, and particularly to methods for expressing and preparing an antibody fusion protein.

### Background

A bispecific antibody refers to an antibody molecule that can recognize two antigens or two epitopes simultaneously, such as bispecific or multi-specific antibodies capable of binding two or more antigens known in the art, which can be obtained in eukaryotic expression systems or in prokaryotic expression systems through cell fusion, chemical modification, and genetic recombination, etc. Similarly, a multi-specific antibody refers to an antibody molecule that can recognize two or more antigens or multiple epitopes at the same time. An immune hybrid protein refers to a protein having one or more specific antibodies and hybridizing with a cytokine, a polypeptide toxin or other biologically active polypeptide molecules.

Pharmacological studies have revealed that most of the complex diseases involve a variety of disease-related signaling pathways. For example, tumor necrosis factor (TNF), interleukin-6 and other pro-inflammatory cytokines simultaneously mediate immune inflammatory diseases, and the proliferation of tumor cells is often caused by abnormal upregulation of multiple growth factor receptors. Blockage of a single signaling pathway is usually limited in efficacy and is prone to cause the development of resistance. In the treatment of tumors, the expression of MHC on the surface of most cancer cells is down-regulated or even absent, causing escaping from immune killing. Bifunctional antibodies can simultaneously bind immune cells, and tumor cells, to localize immune cells to tumors. Therefore, the development of bifunctional antibodies capable of simultaneously binding two different targets and specific antibodies against more than two different targets have long been an important area in the development of new structural antibodies.

An important mechanism of action of bifunctional antibodies is to mediate T cell killing. In recent years, with the deep insight into the immune escape mechanism of cancer cells and the rise of cancer immunotherapy, the research of antibody drugs for activating T cells has received much attention. It is generally believed that effective activation of T cells requires dual signals. The first signal is from the binding of the MHC-antigen complex on the antigen-presenting cell to the T cell receptor TCR-CD3, and the second signal is a non-antigen-specific costimulatory signal produced upon interaction of the T cells with a co-stimulatory molecule expressed by the antigen-presenting cells. The expression of MHC on the surface of most cancer cells is down-regulated or even absent, causing escaping from immune killing. CD3x bifunctional antibodies can bind to surface CD3 molecules on T cells and surface antigens on cancer cells, respectively, thereby shortening the distance between cytotoxic T cells (Tc or CTL) and cancer cells, and directing T cells to directly kill cancer cells, independent of the dual activation signals of T cells (Baeuerle. PA, Cancer Res. 69 (2009) 4941-4944). The unique activation pattern of T cells by CD3x bifunctional antibodies is considered to be a major advantage in its mechanism of action. Multi-specific antibodies can also be used for anti-tumor or other therapeutic purposes by using several different mechanisms.

Another important mechanism of action of bifunctional antibodies is the simultaneous binding to dual targets to block dual signaling pathways. The mechanism has found use in a wide range of applications, including cancer, autoimmune diseases, inhibition of blood vessel growth and anti-infective treatment. For example, the transmembrane tyrosine kinase receptor HER family plays an important regulatory role in cellular physiology and includes HER1 (erbB1, EGFR), HER2 (erbB2, NEU), HER3 (erbB3), HER4 (erbB4), and other members, which are abnormally highly expressed on the surface of many epithelial-derived solid tumor cells, and thus important targets for tumor targeted therapy. The antibodies that get available in market include Herceptin that binds to the HER2D4 domain, Perjeta that binds to the HER2 D2 domain, and Erbitux that binds to HER1/EGFR, which are widely used in the clinical treatment of solid tumors such as breast cancer, stomach cancer and colorectal cancer. Studies have revealed that the homo- or heterodimers of the same or different members from the HER family activate the intracellular signals and promote the cell proliferation and tumor development. Herceptin blocks the homodimerization of the HER2 receptor, but not the heterodimerization of HER2 with other receptors. HER2 and HER3 are the most potent dimeric forms from the HER family that activate the initial oncogenic signaling. Perjeta capable of blocking this dimerization is clinically used in combination with Herceptin, and a better efficacy than that of a single antibody alone is achieved, indicating the clinical effect of dual target blockage (Kristjansdottir. K., Expert Opin biol Ther 10 (2010) 243-250).

Immune cytokines can be synthesized by fusion of antibodies specific for an antigen or target to cytokines, and these immune cytokines have anti-tumor specific activity and are clinically tested (Gillies SD. Immunocytokines: a novel approach to cancer immune therapy. Targeted Cancer Immune Ther 2009:241-56; List T, Neri D. Immunocytokines: a review of molecules in clinical development for cancer therapy. Clin Pharmacol 2013; 5:29-45; List T, Casi G, Neri D.A: Chemically Defined Trifunctional Antibody-Cytokine-Drug Conjugate with Potent Antitumor Activity. Mol Cancer Ther November 2014 13; 2641).

Hybrid proteins have proteins having different functions fused together. Fusion expression of a region (such as FC) of some antibodies and a protein/polypeptide having a particular biological activity or function can produce a biologic drug with a particular clinical effect. For example, Enbrel (or etanercept), a leading drug that has been sold for many years in the international market, is a hybrid protein of a soluble portion of a tumor necrosis factor receptor and an FC fragment of an antibody. There are many kinds of such hybrid proteins available in the market, indicating that hybrid proteins have wide application value. Currently, methods for preparing hybrid proteins comprise expression of recombinant DNA merely in a host cell (prokaryotic or eukaryotic), which causes great limitation on structures.

Since the monoclonal antibody was initially prepared by Kohlor using hybridoma technology in 1957, it has shown broad application prospects in medical research and clinical diagnosis and treatment of diseases. For a long time, efforts have been made to use monoclonal antibodies to treat a variety of diseases, such as tumors, autoimmune diseases, and so on. However, the effect is sometimes undesirable if monoclonal antibodies are used alone. In order to achieve a more potent therapeutic effect, some cytotoxic proteins are bound with monoclonal antibodies to form an "immunotoxin" capable of selectively killing cells bound. Therefore, the ammunition depot for targeted treatment of the diseases is equipped with new ammunitions.

Immunotoxins (ITs) are protein molecules produced by binding a protein having a guiding function to a toxin protein. The portion with targeting function is mainly responsible for targeting the specific binding of the immunotoxin protein molecule to the target cell, and the toxin protein portion mainly plays a role in killing the cell.

From the perspective of the development and evolution of immunotoxins, the preparation and production of immunotoxins mainly include chemical coupling and recombinant expression. The preparation of immunotoxins by chemical coupling includes the preparation of antibodies and toxins separately, followed by chemical coupling to form immunotoxins. Chemical coupling has the disadvantages of low coupling efficiency, high production cost, and poor product uniformity due to the presence of a large number of sites on the protein where coupling reaction may occur. Moreover, the chemical bond produced by coupling tends to degrade during circulation in the body, which causes the release of naked toxins, leading to non-specific toxicity and high risk of toxic side effects, and naked antibodies produced by degradation may block the antigen, resulting in poor therapeutic effects. With the development of genetic engineering, the preparation and production of immunotoxins has entered a new era. Recombinant DNA technology is used to fuse a gene encoding a targeting functional polypeptide to a gene encoding a toxin polypeptide and to express it in an appropriate expression system. The immunotoxins produced by this technical solution are called genetically engineered immunotoxin, which has greatly improved product uniformity and stability compared with the immunotoxins produced by chemical coupling, making the mass production of immunotoxins possible. However, the production of immunotoxins by genetic engineering method also suffers from limitations. The expression of fusion gene is restricted to a single host, and there is a contradiction that the targeting portion and the toxic portion constituting the immunotoxin often require different host expression environments, which often leads to the disadvantages of inability to achieve a good production, yield, and purity through expression of an immunotoxin in a single host cannot, and consequently increased cost. For example, E. coli expression systems are currently used to express single-chain antibody immunotoxins. The targeting portion of immunotoxins does not fold well and forms an inclusion body generally in the E. coli expression system, and the refolding of the inclusion body is a very complicated process. In general, the refolding efficiency of proteins is about 20%. The toxin proteins are lethal to eukaryotic cells. If an eukaryotic expression system is used, the toxin proteins may be toxic to host cells. However, many efforts are made by researchers to express immunotoxins in eukaryotic expression systems. For example, Chinese Patent No.: CN1863921 discloses a method for expressing immunotoxins in Pichia pastoris and EF-2 mutant containing Pichia pastoris. Although the immunotoxins have been successfully expressed through excretion in Pichia pastoris and EF-2 mutant containing (toxin-immunized) Pichia pastoris, a low production resulting from a long fermentation cycle is not competitive, compared with the prokaryotic expression system. Moreover, the glycosylation site on the toxin protein may be glycosylated by the host, which may result in the product heterogeneity. A method for expressing immunotoxins in EF-2 mutant containing CHO cells is disclosed, which also suffers from the problems of low expression level, long fermentation cycle, high cost, and risk of potential glycosylation (Protein expression and purification, 2000, 19(2): 304-311).

Split intein is composed of a N-fragment of intein (In) and a C-fragment of intein (Ic). The gene expressing a precursor protein is split in two open reading frames. The split site is inside the intein sequence. The expression genes of the N-extein (En) and the In of the split intein form a fusion gene, and the fusion protein formed after translation is called an N precursor protein. The expression genes of the Ic of the split intein and the C-extein (Ec) form a fusion gene, and the fusion protein produced after translation is called a C precursor protein. The In or Ic of the split intein alone does not have a protein splicing function. However, after translation into a protein, the In in the N precursor protein and the Ic in the C precursor protein recognize and bind to each other by a non-covalent bond to form a functional intein, and thus can catalyze the protein trans-splicing to link two separated exteins (En, EC) with a peptide bond (Ozawa. T., Nat Biotechbol 21 (2003) 287-93). WO 02/46208 (Elusys Therapeutics inc [US]) disclosed a method for preparing bi-specific antibodies by protein trans-splicing.

Protein trans-splicing refers to a protein splicing reaction mediated by a split intein. In this type of splicing process, the In and Ic of the split intein first recognize and bind to each other by a non-covalent bond. After binding, the structure is properly folded, and the split intein with a re-constructed active center completes the protein splicing reaction following a typical protein splicing route, to link the extein on both sides (Saleh.L., Chemical Record 6 (2006) 183 -193).

A wide variety of recombinant bispecific antibody formats have been developed in the recent past, for example, tetravalent bispecific antibodies by fusion of e.g. an IgG antibody format and single chain domains (see, for example, Coloma, MJ, et al, Nature Biotech. 15 (1997) 159-163; WO 2001077342; and Morrison, S., L., Nature Biotech. 25 (2007) 1233-1234). Due to the large difference in structure from the natural antibodies, they cause a strong immune response and have a short half-life after entering the body.

Also several other new formats where the antibody core structure (IgA, IgD, IgE, IgG or IgM) is no longer retained small molecular antibodies such as dia-, tria- or tetrabodies, mini-bodies, several single chain formats (scFv, Bis-scFv), which are capable of binding two or more antigens, have been developed (Holliger, P., et al, Nature Biotech 23 (2005) 1126-1136; Fischer, N., and Léger, O., Pathobiology 74 (2007) 3-14; Shen, J., et al., J. Immunol. Methods 318 (2007) 65-74; and Wu, C., et al., Nature Biotech 25 (2007) 1290-1297). Although linking a core binding region of an antibody to a core binding protein of another antibody by a linker has advantages for the engineering of bispecific antibodies, problems may exist when they are used as a drug, which greatly limit the use thereof as a drug. Indeed, these foreign peptides might elicit an immune response against the linker itself or both the protein and the linker, causing cytokine storms. Furthermore, the flexible nature of these linkers makes them more prone to proteolytic cleavage, potentially leading to poor antibody stability, aggregation, increased immunogenicity, and short half-life. For example, blinatumomab from Amgen has a half-life of only 1.25 hours and needs be administered continuously by a syringe pump for 24 hours to achieve a therapeutic effect, thus greatly limiting the use (Bargou, R and Leo. E., Science 321 (2008)) 974-7). In addition, one may want to retain effector functions of the antibody, such as complement-dependent cytotoxicity (CDC) or antibody dependent cellular cytotoxicity (ADCC) and extended half-life of binding to FcRn (Fc receptor) in vascular endothelium, which are mediated through the Fc region.

Thus ideally, one should aim at developing bispecific antibodies that are very similar in structure to naturally occurring antibodies (like IgA, IgD, IgE, IgG or IgM), and humanized bispecific antibodies and fully human bispecific antibodies with minimal deviation from human antibody sequences.

In 1983, bispecific antibodies that are very similar to natural antibodies were initially produced using the quadroma technology (Milstein, C and A.C. cuello, Nature, 305 (1983) 537-40). In the quadroma technology, two different murine monoclonal hybridoma cell lines are fused, and up to 10 different kinds of antibodies are produced after fusion, only one of which is the desired bispecific antibody. Due to the high similarity between the physical and chemical properties of the mis-paired products and the product of interest and the extremely low content of the product of interest, a sophisticated purification procedure is required (Morrison, S.L., Nature Biotech 25 (2007) 1233-1234). For example, the bispecific antibody Catumaxomab (Removab), which was marketed in Europe in 2009, causes a serious immune storm upon injection into the human body because the antibody is murine derived, which limits its prospects (Framton.JE., Drugs 72 (2012) 1399-410). Similarly, the mispairing of heavy chains and mispairing of light chains can still not be solved by the recombinant gene expression technology.

To solve the problem of mispairing of heavy chains, a "Knobs-into-Holes" theory is proposed, which aims at forcing the pairing of two different antibody heavy chains by introducing mutations into the CH3 domains of an antibody to modify the contact interface. On one CH3, bulky amino acids are mutated into amino acids with short side chains to create "holes". Conversely, amino acids with large side chains are introduced into the other CH3 domain, to create "knobs". By co-expressing two heavy chains and two light chains (which have to be appropriate for both heavy chains), high yields of heterodimer formation (knob-hole) versus homodimer formation ('hole-hole' or 'knob-knob') is observed (Ridgway, J.B., Protein Eng. 9 (1996) 617-621; and WO96/027011). Although this format appears very attractive, no data about use in clinic is currently available. One important constraint of this strategy is that the light chains of the two parent antibodies have to be identical to prevent mispairing of light chains and formation of impurity molecules. For the problem of mispairing of light chains, the binding specificity of an antibody is altered by mutation to form a "Two-in-One" bivalent bispecific antibody, such that the same specific binding domain of the antibody can bind to two antigens. The binding of such an antibody to each target is bivalent. Although desired effects can be obtained in ligating and activating the target, a defect exists in blocking the effect of the antigens. This method requires a large amount of mutations and other genetic engineering means for each two antibody sequences, and is not a simple and general-purpose method (Bostrom, J., Science 323 (2009) 1610-1414; and Schaefer, G., Cancer Cell 20 (2011) 472-486). In addition, the problem of mispairing of light chains can be optimized by the crossmab (hybrid antibody) method. Exchange of some domains in the light chain and heavy chain of one Fab to form a crossmab (hybrid antibody) can be easily achieved. However, the hybrid antibody contains non-naturally occurring domain linked, losing the native antibody structure (Schaefer, W., Pro. Natl. Acad. Sci. USA 108 (2011) 1187-1192).

Genentech uses a method of co-culturing E. coli expressing two half-antibodies respectively to obtain a bispecific antibody. However, the antibody expressed by this method is not glycosylated, which affects the ADCC effect and half-life in blood, thus limiting the possibility for use as a drug (Spiess, C., Nature Biotechnol 31 (2013) 753-758). In order to produce a bispecific antibody having a structure similar to that of a naturally occurring antibody and containing glycosylation modifications, the problem of mispairing of heavy chains and mispairing of light chains is solved by undergoing structural analysis and site-directed mutagenesis at the interface of Fab, and by transient transfection of 293E cells by the "Knobs-into-Holes" technology, with which great improvements are made. However, in this method, a crystal model needs to be established for each antibody to design a suitable mutation screening site, such that the method is not universal for the construction of every bispecific antibody (Levis, SM, Nature Biotechnol 32 (2014) 191-198). In addition, in the cFAE "half-antibody exchange technology", half-antibodies are directed to recombine by introducing mutations in the CH3 region. An antibody is reduced into half antibodies by in vitro reduction, and then the half antibodies are oxidized into an intact antibody, thereby solving the problems of mispairing of heavy chains and mispairing of light chains. However, there will be 5% mispairing that cannot be solved, and the mis-paired products cannot be removed by purification. The presence of impurity components greatly limits the possibility of use of cFAE as a drug (Labrijin, AF, Nature protocol) (2014) 2045-2463).

Efforts have been made to establish a method for the production of bispecific antibodies which have no non-native domains, are highly structurally similar to natural antibodies (IgA, IgD, IgE, IgG or IgM), have an Fc domain and good structural integrity and stability, retain the complement-dependent cytotoxicity (CDC) or antibody dependent cellular cytotoxicity (ADCC), and have increased in-vivo half-life of binding to FcRn (Fc receptor) and reduced immunogenicity. In the method, no linkers are introduced, so the stability of the antibody molecules is improved, and the immune response is reduced in vivo. The method can be used to produce humanized bispecific antibodies and fully human bispecific antibodies having a sequence close to human antibodies, thereby effectively reducing the immune response. The antibodies are produced in a mammalian cell expression system, have glycosylation modifications, have better biological functions, are more stable, and have long half-life in vivo. The mispairing of heavy chains is effectively avoided, and the mispairing rate can be reduced to 0%. The mispairing of light chains is effectively avoided, and the mispairing rate of light chains can be reduced to 0%. The method is a general-purpose method for constructing bispecific antibodies, has no limitations arising from antibody subtypes (IgG, IgA, IgM, IgD, IgE, IgM, and light chains κ and λ), does not require the design of different mutations depending on specific targets, and can be used to construct any bispecific or multi-specific antibodies.

A strategy to produce bispecific constructs using inteteins IN and IC has been described in WO02/46208. **SUMMARY**

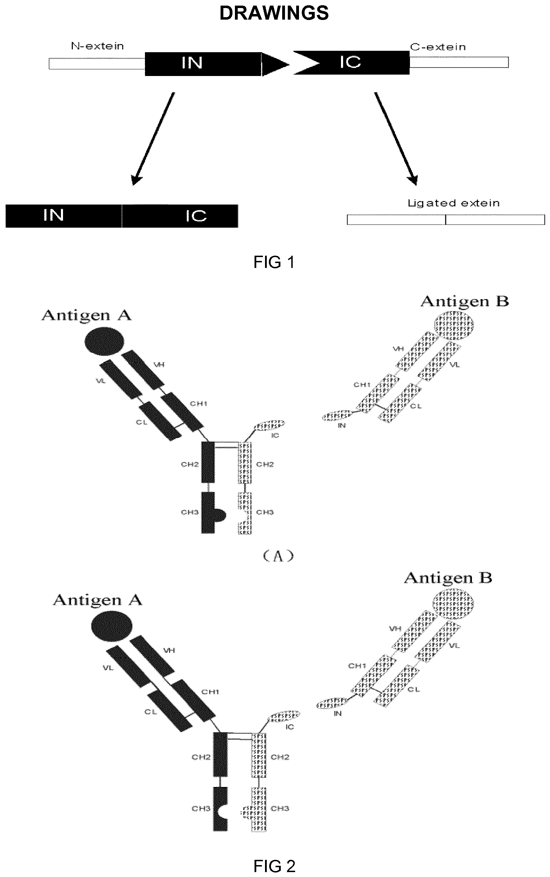

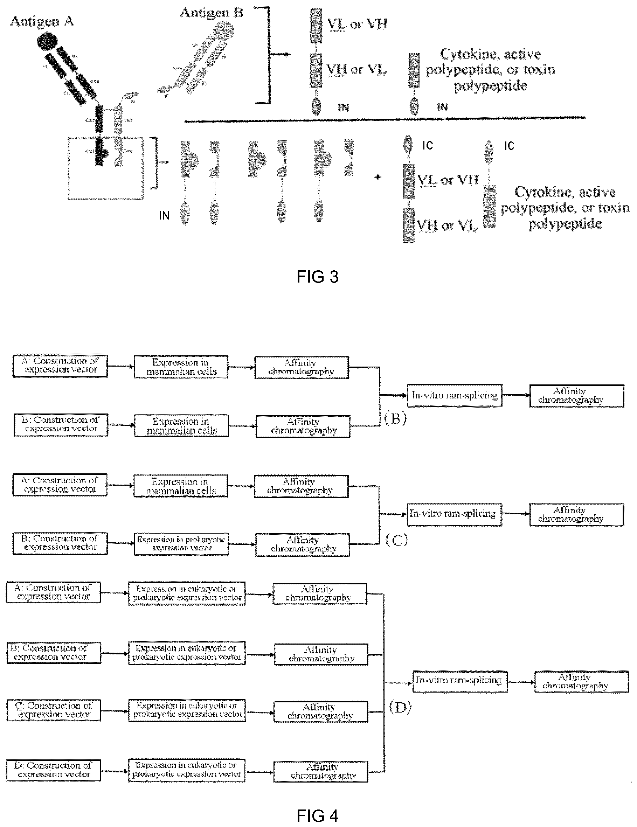

An object of the present disclosure is to provide novel methods for expressing and preparing an antibody fusion protein. Methods for expressing and preparing a polyvalent multi-specific antibody and an immune hybrid protein are also disclosed. In the present disclosure, a bispecific antibody is split into an antigen A binding portion and an antigen B binding portion for the first time, as shown (in Figs. 2A and 2B), which are expressed separately, and then ligated into an intact antibody by protein trans-splicing by a split intein. The portion A comprises a light chain of an antibody A, an intact heavy chain of the antibody A, and an Fc chain having Ic fused to the N terminal. The B comprises a light chain of an antibody B and a VH+CH1 chain of the antibody B having In fused to the C terminal Furthermore, as shown in FIG. 3, in addition to the bispecific antibodies, single chains (including VL and VH specifically binding to a second target), heavy and light chains, cytokines, active polypeptides, toxin polypeptides, and the like may be ligated in the binding region of the antibody molecule by protein/ram-splicing by the Intein in the present disclosure. Single chains (including VL and VH specifically binding to a second target), heavy and light chains, cytokines, active polypeptides, toxin polypeptides, and the like may be ligated to the C terminal of the FC region of the antibody molecule by protein/ram-splicing by the Intein in the present disclosure. The present invention is defined by the appended set of claims.

The objects of the present disclosure are accomplished through the following technical solutions, i.e. a method for expressing and preparing an antibody fusion protein, wherein said antibody fusion protein comprises:
a light chain and a heavy chain of a first antibody that specifically binds to a first antigen; optionally wherein the heavy chain further comprises an IN domain linked to its C-terminus;
a light chain and the VH+CH1 portion of a heavy chain of a second antibody that specifically binds to a second antigen, wherein the C-terminus of the CH1 domain is fused to an Intein-N moiety (IN);
an Fc chain from said second antibody, wherein the N-terminus of the Fc chain is linked to an Intein-C moiety (IC); optionally, wherein said Fc chain further comprises an IN domain linked to its C-terminus;
a knob and a corresponding hole capable of associating with each other, the knob and hole being located in the interface of the CH3 domain of the heavy chain of the first antibody and of the CH3 domain of the Fc chain or vice versa;
wherein the method comprises the following steps:
   A1: obtaining and amplifying the coding sequences of a "portion A antibody", said portion A antibody comprises said light chain and said heavy chain of said first antibody, and said Fc chain of said second antibody, in which the Intein-C moiety (IC) is fused to the N-terminus of said Fc chain;
   A2: constructing an eukaryotic or prokaryotic expression vector by whole-gene synthesis, wherein the vector comprises the coding sequences amplified in step A1 and obtaining the portion A antibody by expressing the vector via transient transfection or stable transfection; and
   B1: obtaining and amplifying the coding sequence of a "portion B antibody", said portion B antibody comprises said light chain and said VH+CH1 chain of said second antibody, in which the C-terminus of said VH+CH1 chain is fused to the Intein-N moiety (IN);
   B2: constructing an eukaryotic or prokaryotic expression vector by whole-gene synthesis, wherein the vector comprises the coding sequences amplified in step B1 and obtaining the portion B antibody by expressing the vector via transient transfection or stable transfection; and
   D: obtaining the antibody fusion protein by subjecting the portion A antibody, the portion B antibody to protein trans-splicing in vitro.

In a preferred embodiment of the present invention, the inventive method further comprises the following steps:
C1: obtaining and amplifying the coding sequence of a "portion C antibody", said portion C antibody comprises a single chain third antibody, in which IC is fused the N-terminus of said third antibody; and the IC is for binding to the further C-terminal IN of the heavy chain of said first antibody; or, the IC is for binding to the further C-terminal IN of the Fc chain of said second antibody;
   and/or,
C2: obtaining and amplifying the coding sequence of a "portion D antibody", said portion D antibody comprises a single chain fourth antibody, in which IC is fused to the N-terminus of said fourth antibody; and the IC is for binding to the further C-terminal IN of the heavy chain of said first antibody; or, the IC is for binding to the further C-terminal IN of the Fc chain of said second antibody; and
D: obtaining the antibody fusion protein by subjecting the portion A antibody, the portion B antibody, the portion C antibody, and/or the portion D antibody to protein trans-splicing in vitro.

The method may further comprise the following steps:
C1: obtaining and amplifying the coding sequence of a protein molecule, and IC is fused to N-terminus of the protein molecule; and the IC is for binding to the further C-terminal IN of the heavy chain of said first antibody; or, the IC is for binding to the further C-terminal IN of the Fc chain of said second antibody;
   and/or,
C2: obtaining and amplifying the coding sequence of a protein molecule, and IC is fused to N-terminus of the protein molecule; and the IC is for binding to the further C-terminal IN of the heavy chain of said antibody; or, the IC is for binding to the further C-terminal IN of the Fc chain of said second antibody;
said protein molecule comprises cytokines, toxin polypeptides or active polypeptides; and
D: obtaining the antibody fusion protein by subjecting the portion A antibody, the portion B antibody, the protein molecule with the fused IC to protein trans-splicing in vitro.

Preferably, a knob is formed at an interface of the CH3 domain in the first heavy chain, which can be located in a hole formed at an interface of the CH3 domain in the Fc chain of the second heavy chain having Ic fused to the N terminal.

Preferably, the threonine at position 366 in the CH3 domain of the first heavy chain is mutated to tryptophan to form the knob; and in the CH3 domain of the Fc chain of the second heavy chain having Ic fused to the N terminal, the threonine at position 366 is mutated to serine, the leucine at position 368 is mutated to alanine, and the tyrosine at position 407 is mutated to valine, to form the hole.

Preferably, the serine at position 354 in the CH3 domain of the first heavy chain is mutated to cysteine; and the tyrosine at position 349 in the CH3 domain of the Fc chain of the second heavy chain having Ic fused to the N terminal is mutated to cysteine.

Preferably, a hole is formed at an interface of the CH3 domain in the first heavy chain, in which a knob formed at an interface of the CH3 domain in the Fc chain of the second heavy chain having Ic fused to the N terminal can be located.

Preferably, in the CH3 domain of the first heavy chain, the threonine at position 366 is mutated to serine, the leucine at position 368 is mutated to alanine, and the tyrosine at position 407 is mutated to valine, to form the hole; and in the CH3 domain of the Fc chain of the second heavy chain having Ic fused to the N terminal, the threonine at position 366 is mutated to tryptophan, to form the knob.

Preferably, the tyrosine at position 349 in the CH3 domain of the first heavy chain is mutated to cysteine; and the serine at position 354 in the CH3 domain of the Fc chain of the second heavy chain having Ic fused to the N terminal is mutated to cysteine.

In the present disclosure, in order to improve the binding stability of the CH3 regions, the S (serine) at position 354 on the "knob" chain is mutated to C (cysteine), and the Y (tyrosine) at position 349 on the "hole" chain is mutated to C (cysteine), to enhance the stability between heavy chains by introducing a pair of inter-heavy chain disulfide bonds.

Preferably, in Step A2 and B2, the eukaryotic expression vector is a mammalian cell. The mammalian cell expression vector is constructed by whole-gene synthesis as follows. Specifically, whole-gene chemical synthesis is performed according to the designed split gene sequences. Restriction endonuclease cleavage sites are added at the two sides of the start codon and the stop codon by PCR. The genes are respectively inserted into a mammalian cell expression vector containing CMV promoter, the sub-clones are sequenced, and the plasmids are extracted.

Preferably, in Step A2 and B2, the expression is expression by a mammalian cell expression system.

Preferably, in Step A2 and B2, the mammalian cell is 293E, 293F or CHO cells.

Preferably, the product expressed in Step A2 and B2is obtained through purification by ProteinL affinity chromatography or by ProteinA/G chromatography; and the fusion protein in Step D is obtained through purification by ProteinA/G chromatography.

Preferably, in Step A2 and B2, the transient transfection is transient transfection of 293-E, 293-F or CHO cells, and the steady transfection is steady transfection of CHO cells.

Preferably, in Step D, the in-vitro trans-splicing is in-vitro trans-splicing mediated by a split intein in the presence of a sulfhydryl compound, i.e., a trans-splicing reaction of the split intein.

Preferably, the in-vitro trans-splicing occurs at a temperature of 4-37°C and is continued for 5-120 min, and the concentration of the sulfhydryl compound is 0.05-2 mM.

Preferably, in Step D, a step of purifying the spliced product by affinity chromatography is further included.

Further disclosed is a method for expressing and preparing an immune hybrid protein, which comprises the following steps:
A1: splitting an expressed sequence of the immune hybrid protein, to obtain a protein molecule and a portion A antibody or the portion A antibody and a portion B antibody, where the portion A antibody comprises a first light chain, a first heavy chain, and an Fc chain of a second heavy chain, in which the Fc chain has IN fused to the C terminal; the portion B antibody comprises a second single chain having In fused to one end; and the protein molecule has IC fused to one end, where the first light chain and the first heavy chain are a first light chain and a first heavy chain of an antibody that specifically binds to a first antigen; and the second heavy chain and the second single chain are a second heavy chain and a second single chain of an antibody that specifically binds to a second antigen;
A2: constructing an eukaryotic or prokaryotic expression vector by whole-gene synthesis, and expressing and preparing the portion A antibody or the portion A antibody and the portion B antibody by transient transfection or steady transfection; and
A3: subjecting the portion A antibody and the protein molecule to protein trans-splicing in vitro, or subjecting the portion A antibody and the portion B antibody to protein trans-splicing in vitro, to obtain the immune hybrid protein.

Preferably, the protein molecule includes cytokines, toxin polypeptides or active polypeptides.

Also disclosed is a method for expressing and preparing an immune hybrid protein, which comprises the following steps:
B1: splitting an expressed sequence of the immune hybrid protein, to obtain a protein molecule, a portion A antibody, and a portion B antibody, where the portion A antibody comprises a first light chain, a first heavy chain, and an Fc chain of a second heavy chain, in which the Fc chain has IN fused to the C terminal; the portion B antibody comprises a second light chain and a VH+CH1 chain of the second heavy chain, in which the VH+CH1 chain has In fused to the C terminal; the first light chain and the first heavy chain are a first light chain and a first heavy chain of an antibody that specifically binds to a first antigen; the second light chain and the second heavy chain are a second light chain and a second heavy chain of an antibody that specifically binds to a second antigen; the protein molecule has ICfused to one end, and at least one of the Fc chain of the second heavy chain and the Fc chain of the first heavy chain has IN fused to the C terminal;
B2: constructing an eukaryotic or prokaryotic expression vector by whole-gene synthesis, and expressing and preparing the portion A antibody and the portion B antibody by transient transfection or steady transfection; and
B3: subjecting the portion A antibody, the portion B antibody, and the protein molecule to protein trans-splicing in vitro, to obtain the immune hybrid protein.

Preferably, the protein molecule includes cytokines, toxin polypeptides or active polypeptides.

Also disclosed is a method for preparing an immunotoxin, which comprises the following steps:
Step 1: splitting a structural sequence of the target immunotoxin into a structure I and a structure II, where the structure I is an antibody or a fragment thereof; and the structure II is a toxin portion;
Step 2: expressing the structure I and the structure II respectively; and
Step 3: ligating the structure I and the structure II by protein trans-splicing by a split intein, to obtain the target immunotoxin.

Furthermore disclosed is a method for preparing a cytokine-antibody fusion protein, which comprises the following steps:
Step 1: splitting a structural sequence of the target cytokine-antibody fusion protein into a structure I and a structure II, where the structure I is an antibody or a fragment thereof; and the structure II is a cytokine portion;
Step 2: expressing the structure I and the structure II respectively; and
Step 3: ligating the structure I and the structure II by protein trans-splicing by a split intein, to obtain the target cytokine-antibody fusion protein.

Finally, also disclosed is a method for preparing an ADC antibody, which comprises the following steps:
Step 1: splitting a structural sequence of the target ADC antibody into a structure I and a structure II, where the structure I is an antibody or a fragment thereof; and the structure II is a compound portion;
Step 2: expressing the structure I and the structure II respectively; and
Step 3: ligating the structure I and the structure II by protein trans-splicing by a split intein, to obtain the target ADC antibody.

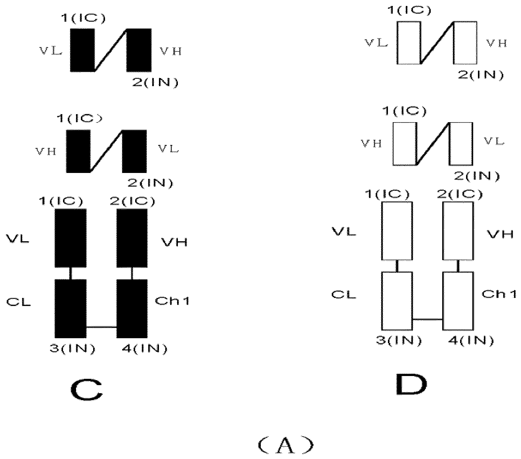

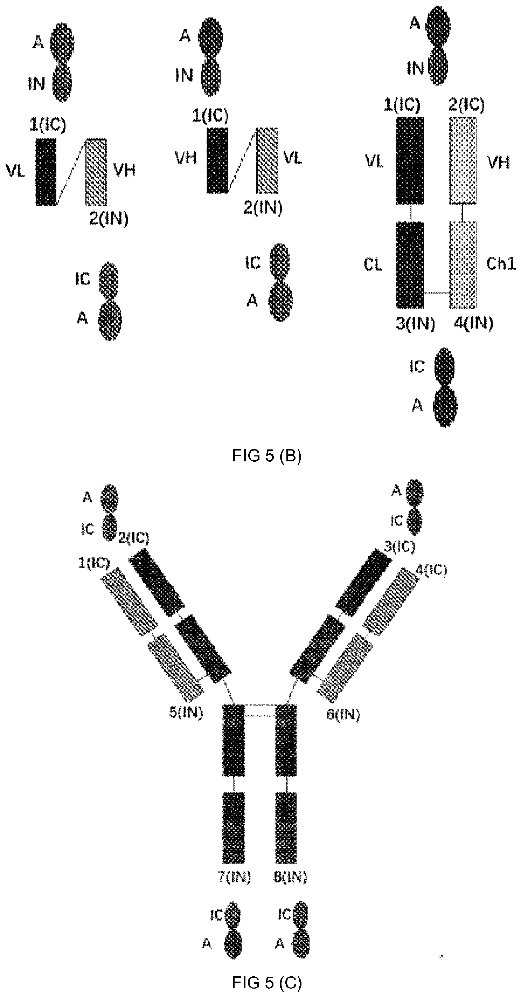

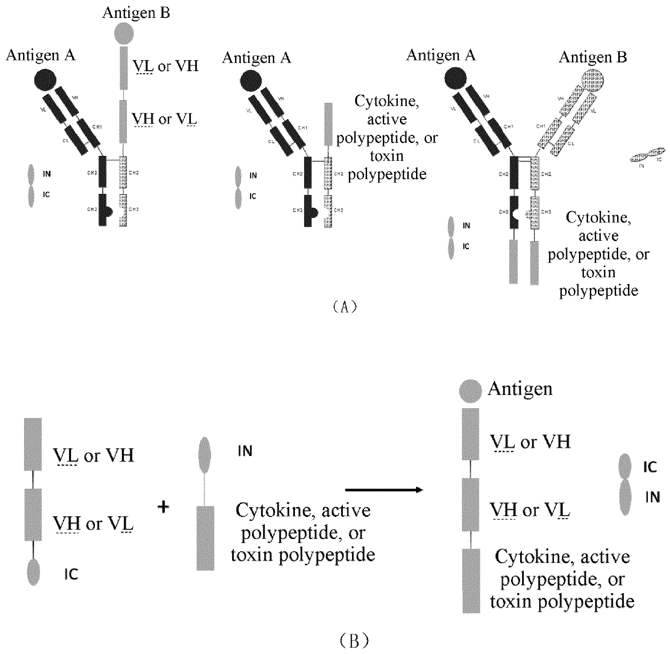

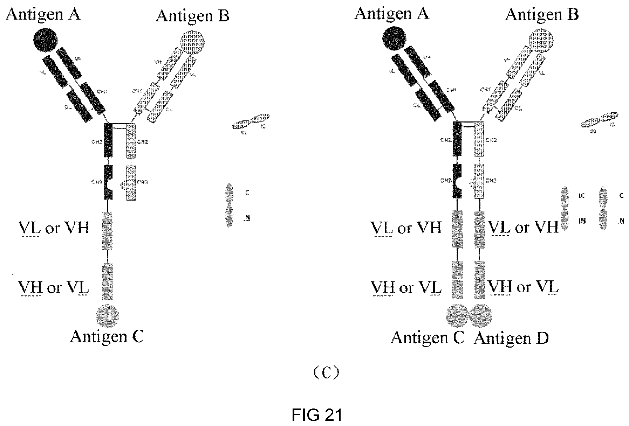

Notably:
1) The bispecific antibodies expressed and prepared by the method of the present disclosure have no non-native domains, are highly structurally similar to natural antibodies (IgA, IgD, IgE, IgG or IgM), have an Fc domain and good structural integrity and stability, retain the complement-dependent cytotoxicity (CDC) or antibody dependent cellular cytotoxicity (ADCC), and have increased in-vivo half-life of binding to FcRn (Fc receptor) and reduced immunogenicity.
2) No linkers are introduced, so the stability of the antibody molecules is improved, and the immune response is reduced in vivo.
3) The method can be used to produce humanized bispecific antibodies and fully human bispecific antibodies having a sequence close to human antibodies, thereby effectively reducing the immune response.
4) The antibodies are produced in a mammalian cell expression system, have glycosylation modifications, have better biological functions, are more stable, and have long half-life in vivo.
5) The mispairing of heavy chains is effectively avoided, and the mispairing rate can be reduced to 0%; and the mispairing of light chains is effectively avoided, and the mispairing rate of light chains can be reduced to 0%, thereby improving the efficiency of product purification and ensuring that the final product is free of contamination from mis-paired impurities.
6) The method of the present disclosure is a general-purpose method for constructing bispecific antibodies, has no limitations arising from antibody subtypes (IgG, IgA, IgM, IgD, IgE, IgM, and light chains κ and λ), does not require the design of different mutations depending on specific targets, and can be used to construct any bispecific antibodies.
7) The method of the present disclosure can also be used to construct a bispecific antibody in which the Fc fragment is defective, for example, only a portion of the CH2 region in the Fc region is retained, or an intact CH2 region and a portion of the CH3 region are retained.
8) Moreover, the method of the present disclosure can also be used to construct a bispecific antibody in which a Fab fragment remains, for example the portion A is Scfv, and the portion B is Fab; the portion A is Fab, and the portion B is Scfv; or the portion A is Scfv and the portion B is Scfv; and to construct a bispecific antibody retaining an intact Fc region or a defective Fc region.
9) The present disclosure is applicable to the construction of a small molecule antibody fragment of the type indicated by Group C and a small molecule fragment antibody of the type indicated by Group D in FIG. 5 by protein trans-splicing mediated by split intein.
10) The present disclosure is applicable to any groups of combinations shown in FIG. 21, to prepare a polyvalent, multi-specific (including bivalent, bispecific) antibody; and to the preparation of immune hybrid proteins (including immunocytokines, and immunotoxins, etc.). In each case, protein trans-splicing mediated by split intein is employed to prepare multi-specific (including bispecific) antibodies in vitro. The product contains no mis-paired impurities, which improves the efficiency of separation and purification.

### BRIEF DESCRIPTION OF THE DRAWINGS

Other features, objects, and advantages of the present disclosure will become apparent upon reading the detailed description of non-limiting embodiments that follow with reference to the accompanying drawings.
FIG. 1 is a schematic view showing the protein trans-splicing mediated by a split intein;
FIG. 2 is a schematic view showing the splitting of a bispecific antibody, in which A is a schematic view showing the splitting of the bispecific antibody into a portion A antibody having a knob-type heavy chain and a hole-type Fc chain, and a portion B antibody; and B is a schematic view showing the splitting of the bispecific antibody into a portion A antibody having a hole-type heavy chain and a knob-type Fc chain, and a portion B antibody;
FIG. 3 is a schematic view showing a hybrid protein having an antibody portion (including hybrid cytokines, toxins, and single chains etc.);
FIG. 4 is a flow chart showing the preparation of bispecific antibodies, where A shows the splicing of fragments expressed in two mammalian cells, B shows the splicing of fragments expressed in prokaryotic cells, C shows the splicing of two fragments in which one is expressed in a mammalian cell and the other is expressed in a prokaryotic cell, and D shows the splicing of four fragments which are expressed either in mammalian cells or in prokaryotic cells;
FIG. 5A is a schematic view showing the construction of a fragment-type bispecific antibody; B is a schematic view showing the construction of an immune hybrid protein of an antibody fragment; and C is a schematic view showing the construction of an immune hybrid protein of a full-length antibody;
FIG. 6 is a schematic view showing a light chain of a portion A antibody;
FIG. 7 is a schematic view showing a knob-type heavy chain of the portion A antibody;
FIG. 8 is a schematic view showing a hole-type Fc chain of the portion A antibody;
FIG. 9 is a schematic view showing a heavy chain of a portion B antibody and IN;
FIG. 10 is a schematic view showing a light chain of the portion B antibody;
FIG. 11 is a schematic view showing a hole-type heavy chain of a portion A antibody;
FIG. 12 is a schematic view showing a knob-type Fc chain of the portion A antibody;
FIG. 13 is an SDS-PAGE electrophoretogram of a purified product co-transfected with three expression vectors of a portion A antibody of a bispecific antibody.
FIG. 14 is an SDS-PAGE electrophoretogram of a purified product co-transfected with a three-expression vector of a portion B antibody of a bispecific antibody.
FIG. 15 is a schematic view showing the splicing (type I) of a portion A antibody and a portion B antibody mediated by a split intein;
FIG. 16 is a schematic view showing the splicing (type II) of a portion A antibody and a portion B antibody mediated by a split intein;
FIG. 17 is a schematic view showing the trans-splicing into a bispecific antibody induced by a split intein at various DTT concentrations (mM);
FIG. 18 is a schematic view showing the trans-splicing into a bispecific antibody induced by a split intein at various temperatures (°C);
FIG. 19 is a schematic view showing the trans-splicing into a bispecific antibody induced by a split intein at various reaction times (min);
FIG. 20 is an SDS-PAGE electrophoretogram of a bispecific antibody purified by ProteinA affinity chromatography; and
FIG. 21 is a schematic view showing products prepared through methods for preparing a multi-specific antibody and an immune hybrid protein, in which A shows a product group A prepared through a method for preparing a multi-specific antibody and an immune hybrid protein (bispecific antibodies, antibody-directed immunotoxins, and other products); B shows a product group B prepared through a method for preparing a multi-specific antibody and an immune hybrid protein (hybrid proteins of antibody fragments with cytokines or toxins); and C shows a product group C prepared through a method for preparing a multi-specific antibody and an immune hybrid protein (bi- to tetra-specific antibodies, immune hybrid proteins, and other products).

### DETAILED DESCRIPTION

The present invention is defined by the appended claims. The technical disclosure set out below may in some respects go beyond the scope of the claims. Elements of the disclosure which do not fall within the scope of the claims are provided for information.

Terms used in the present disclosure are defined below.

Antibody refers to an intact monoclonal antibody. The intact antibody consists of two pairs of "light chain" (LC) and "heavy chain" (HC) (the light chain/heavy chain pair is abbreviated as LC/HC). The light and heavy chains of the antibody are polypeptides consisting of several domains. In intact antibodies, each heavy chain includes a heavy chain variable region (abbreviated as HCVR or VH) and a heavy chain constant region. The heavy chain constant region includes heavy chain constant domains CH1, CH2 and CH3 (antibody types IgA, IgD, and IgG) and, optionally, heavy chain constant domain CH4 (antibody types IgE and IgM). Each light chain includes a light chain variable domain VL and a light chain constant domain CL. The structure of a naturally occurring intact antibody, i.e., an IgG antibody, is shown, for example, in FIG. 1. The variable domains VH and VL can be further subdivided into hypervariable regions called complementarity determining regions (CDRs), with more conserved regions called framework regions (FR) distributed between them. VH and VL each consist of three CDRs and four FRs, arranged from the amino terminal to the carboxy terminal in the following order: FR1, CDR1, FR2, CDR2, FR3, CDR3, and FR4 (Janeway, CA, Jr. et al., Immunobiology, 5th Edition, Garland Publishing (2001); and Woof J, Burton D Nat Rev Immunol 4 (2004) 89-99). The two pairs of heavy and light chains (HC/LC) are capable of specifically binding to the same antigen. Thus, the intact antibody is a bivalent, monospecific antibody. The "antibody" includes, for example, a mouse antibody, a human antibody, a chimeric antibody, a humanized antibody, and a genetically engineered antibody (variant or mutant antibody), provided that their specific characteristics are retained. Human or humanized antibodies are particularly preferred, especially recombinant human or humanized antibodies. There are five types of heavy chains in mammalian antibodies, which are represented by Greek letters: α, δ, ε, γ, and µ (Janeway, CA, Jr., et al., Immunobiology, 5th Edition, Garland Publishing (2001)). The types of heavy chains present define the types of antibodies. These chains are present in IgA, IgD, IgE, IgG, and IgM antibodies, respectively (Rhoades RA, Pflanzer RG (2002). Human Physiology, 4th Edition, Tom Thomson Learning). Different heavy chains vary in size and composition. Alpha and gamma types contain approximately 450 amino acids, while µ and ε type have approximately 550 amino acids. Each heavy chain has two regions, that is, a constant region and a variable region. The constant regions are identical in all antibodies of the same isotype, but differ in antibodies of different isotypes. The heavy chains γ, α and δ have a constant region consisting of three constant domains CH1, CH2 and CH3 (on a line) and a hinge region for increasing the flexibility (Woof, J., Burton D Nat Rev Immunol 4 (2004) 89-99). The heavy chains µ and ε have a constant region consisting of four constant domains CH1, CH2, CH3 and CH4 (Janeway, CA, Jr., et al., Immunobiology, 5th Edition, Garland Publishing (2001)). The variable regions of the heavy chain vary in antibodies produced by different B cells, but are identical in all antibodies produced by a single type of B cells or B cell clone. The variable region of each heavy chain is approximately 110 amino acids in length and consists of a single antibody domain. In mammals, there are only two types of light chains, called λ and κ. The light chain has two consecutive domains: a constant domain CL and a variable domain VL. The approximate length of the light chain is 211-217 amino acids. Preferably, the light chain is a kappa light chain and the constant domain CL is preferably Cκ.

The Fc portion of an antibody is a term well known to the skilled artisan and is defined based on the cleavage of the antibody with papain. An antibody according to the present disclosure comprises, for example, an Fc portion, preferably a human derived Fc portion and preferably all other portions of a human constant region. The Fc portion of the antibody is directly involved in complement activation, C1q binding, C3 activation and Fc receptor binding. Although the effect of an antibody on the complement system depends on specific conditions, binding to C1q is caused by specific binding sites in the Fc portion. Such binding sites are known in the art and are described, for example, in Lukas, TJ, et al., J. Immunol. 127 (1981) 2555-2560; Brunhouse, R., and Cebra, JJ., Mol. Immunol. 16 (1979) 907-917; Burton, DR, et al., Nature 288 (1980) 338-344; Thommesen, JE, et al., Mol. Immunol.) 37 (2000) 995-1004; Idusogie, EE, et al., J. Immunol. 164 (2000) 4178-4184; Hezareh, M., et al., J. Virol. 75 (2001) 12161-12168; and Morgan, A., et al., Immunology 86 (1995) 319-324; and EP 0 307 434. The binding sites are, for example, L234, L235, D270, N297, E318, K320, K322, P331 and P329 (in accordance with Kabat's EU catalog number). Antibodies of subtypes IgG1, IgG2 and IgG3 typically exhibit the capabilities of complement activation, C1q binding and C3 activation, whereas IgG4 does not activate the complement system, does not bind to C1q and does not activate C3.

Humanized antibody refers to an antibody in which the frameworks or "complementarity determining region" (CDRs) have been modified to include CDRs of immunoglobulin that differ in specificity compared to the specificity of the parent immunoglobulin. For example, murine CDRs are grafted into the framework regions of human antibodies to produce "humanized antibodies." (Riechmann, L., et al., Nature 332 (1988) 323-327; and Neuberger, M.S., et al., Nature 314 (1985) 268-270).

Human antibodies include antibodies having variable and constant regions derived from sequences of human immunoglobulin.

Recombinant human antibodies refer to all human antibodies prepared, expressed, produced or isolated by recombination, such as antibodies isolated from host cells, such as NS0 or CHO cells, or antibodies isolated from transgenic animals (e.g., mice) with human immunoglobulin genes, or antibodies expressed by a recombinant expression vector transfected into a host cell. The recombinant human antibodies have a variable region and a constant region in a rearranged pattern.

The variable region domain (the light chain (VL) variable region, and the heavy chain (VH) variable region) is each pair of light and heavy chains that are directly involved in the binding of an antibody to an antigen. The human light and heavy chain variable domains have the same general structure and each domain comprises four framework regions (FR), which have a sequence that is generally conserved, and are linked through 3 "hypervariable regions" (or Complementarity determining regions, CDRs). The framework regions take a beta-sheet conformation and the CDRs can form a loop that joins the beta-sheet structure. The CDRs in each chain maintain their three-dimensional structure through the framework regions and form an antigen binding site with the CDRs from the other chain.

Bivalent bispecific antibody refers to an antibody as described above, where each of the two pairs of heavy and light chains (HC/LC) specifically binds to a different antigen, i.e., a first heavy chain and a first light chain (derived from an antibody against an antigen A) specifically bind to the antigen A, and a second heavy chain and a second light chain (derived from an antibody against an antigen B) specifically bind to the antigen B. The bivalent bispecific antibody can simultaneously specifically bind to two and no more than two different antigens, in contrast to a monospecific antibody capable of binding only one antigen on the one hand and a tetravalent tetra-specific antibody capable of simultaneously binding to four antigen molecules on the other hand, for example.

Split intein is composed of a N-fragment of intein (In) and a C-fragment of intein (Ic). The gene expressing a precursor protein is split in two open reading frames. The split site is inside the intein sequence. The expression genes of the N-extein (En) and the In of the split intein form a fusion gene, and the fusion protein formed after translation is called an N precursor protein. The expression genes of the Ic of the split intein and the C-extein (Ec) form a fusion gene, and the fusion protein produced after translation is called a C precursor protein. The In or Ic of the split intein alone does not have a protein splicing function. However, after protein translation, the In in the N precursor protein and the Ic in the C precursor protein recognize and bind to each other by a non-covalent bond to form a functional intein, and thus can catalyze the protein trans-splicing to link two separated exteins (En, EC) with a peptide bond (Ozawa. T., Nat Biotechbol 21 (2003) 287-93).

Protein trans-splicing refers to a protein splicing reaction mediated by a split intein. In this type of splicing process, the In and Ic of the split intein first recognize and bind to each other by a non-covalent bond (FIG. 1). After binding, the structure is properly folded, and the split intein with a re-constructed active center completes the protein splicing reaction following a typical protein splicing route, to link the extein at both sides (Saleh. L., Chemical Record 6 (2006) 183 -193).

IN refers to an N-fragment of a split intein alone.

IC refers to a C-fragment of a split intein alone.

Transient transfection is one of the ways to introduce DNA into eukaryotic cells. In transient transfection, a recombinant DNA is introduced into a cell line with high transfection potential to obtain a transient but high level of expression of the gene of interest. The transfected DNA does not have to be integrated into the chromosome of the host, the transfected cells can be harvested in a shorter time than stable transfection, and the expression of the gene of interest in the cell culture supernatant is detected.

The present disclosure relates to methods for expressing and preparing a novel polyvalent multi-specific antibody and an immune hybrid protein. In the present disclosure, a bispecific antibody is split into an antigen A binding portion and an antigen B binding portion for the first time, as shown (in Figs. 2A and 2B), which are expressed separately, and then ligated into an intact antibody by protein trans -splicing by a split intein. The portion A comprises a light chain of an antibody A, an intact heavy chain of the antibody A, and an Fc chain having Ic fused to the N terminal. The B comprises a light chain of an antibody B and a VH+CH1 chain of the antibody B having In fused to the C terminal In the present disclosure, the trans-splicing function of the split intein is combined with the construction of bispecific antibodies for the first time, and portion A and B antibodies expressed and purified separately are linked to form an intact antibody by means of the trans-splicing function of the split intein. This kind of specific antibodies are similar in structure to naturally occurring antibody molecules, thereby avoiding the instability of antibody molecules due to structural differences and the high immunogenicity in vivo. Firstly, an expressed sequence of the obtained antibody is analyzed and split, a mammalian cell expression vector is constructed by whole gene synthesis, and the purified vector is transiently transfected into mammalian cells such as 293E, 293F, and CHO, etc., or stably transfected into mammal cells such as CHO. The fermentation liquors are separately collected and purified by proteinL affinity chromatography. The purified portions A and B are subjected to trans-splicing in vitro, and the spliced product is purified by proteinA affinity chromatography to obtain a relatively pure bispecific antibody. The process flow is shown in FIG. 4.

The method of the present disclosure can also be used to construct a bispecific antibody in which the Fc fragment is defective, for example, only a portion of the CH2 region in the Fc region is retained, or an intact CH2 region and a portion of the CH3 region are retained. In addition, the method is useful in the linkage of any two types of antibody fragments into a novel bispecific antibody. As shown in (FIG. 5), any form of an antibody fragment of a portion C can be trans-spliced with any form of an antibody fragment of a portion D by the split intein.

The method for expressing and preparing a hybrid protein of a novel bivalent bispecific antibody provided in the present disclosure comprises the following steps

### 1. Construction of expression vector

For the construction of expression vectors, general information about the nucleotide sequences of light and heavy chains of human immunoglobulin is provided in Kabat, EA, et al., Sequences of Proteins of Immunological Interest, 5th Edition, Public Health Services, National Institutes of Health, Bethesda, MD. (1991) and in the drugbank database. The amino acids in the antibody chain are numbered and referenced according to the EU numbering (Edelman, G.M., et al., Proc.Natl.Acad.Sci.USA 63(1969)78-85; Kabat, E.A., et al., Sequences of Proteins of Immunological Interest, 5th Edition, Public Health Service, National Institutes of Health, Bethesda, MD. (1991)). The desired gene segments are prepared by oligonucleotides prepared through chemical synthesis. The 600-1800 bp long gene segment is assembled by annealing and ligation of PCR-amplified oligonucleotides, and then cloned into an expression vector via the indicated restriction sites such as KpnI/BamHI. The DNA sequence of the subcloned gene fragment is verified by DNA sequencing. Infomax's VectorNTI Advance suite version 8.0 is used for sequence construction, mapping, analysis, annotation, and description.

1.1. In order to solve the problem of mispairing of heavy chains, "Knobs-into-Holes" is introduced and the VH and CH1 regions of one heavy chain are removed and IC (C-fragment of the split intein) is fused to the N-hinge region of CH2. Thus, the heavy-chain homodimer component formed by the heavy chain that cannot be purified and removed is completely prevented. In order to introduce the "Knobs-into-Holes" structure, (threonine) at position 366 in a CH3 region is mutated to W (tryptophan) to form a "Knobs" structure. T (threonine) at position 366 in a CH3 region of another heavy chain is mutated to S (serine), L (leucine) at position 368 is mutated to A (alanine), and Y (tyrosine) at position 407 is mutated to V (valine), to form a "Holes" structure. In addition, in order to enhance the binding stability of the CH3 regions, S (serine) at position 354 of the "Knobs" chain is mutated to C (cysteine), and Y (tyrosine) at position 349 on the "Holes" chain is mutated to C (cysteine) to enhance the stability between heavy chains by introducing a pair of inter-heavy chain disulfide bonds.

1.2. In order to introduce the split intein, the heavy chain of an antibody B is split into an Fc region and a VH+CH1 region in the heavy chain hinge region of the antibody B, IN (N-fragment of the split intein) is fused to the C-terminal of the CH1 region, and IC (C-fragment of the split intein) is fused to the N terminal of CH2.

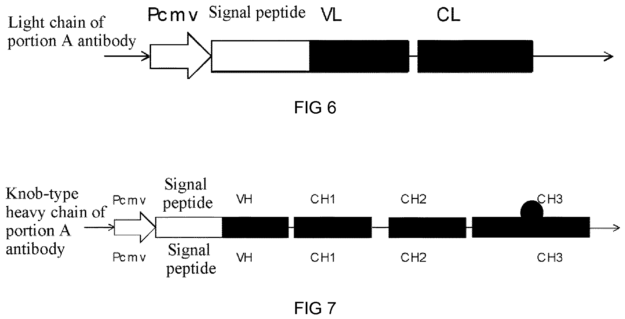

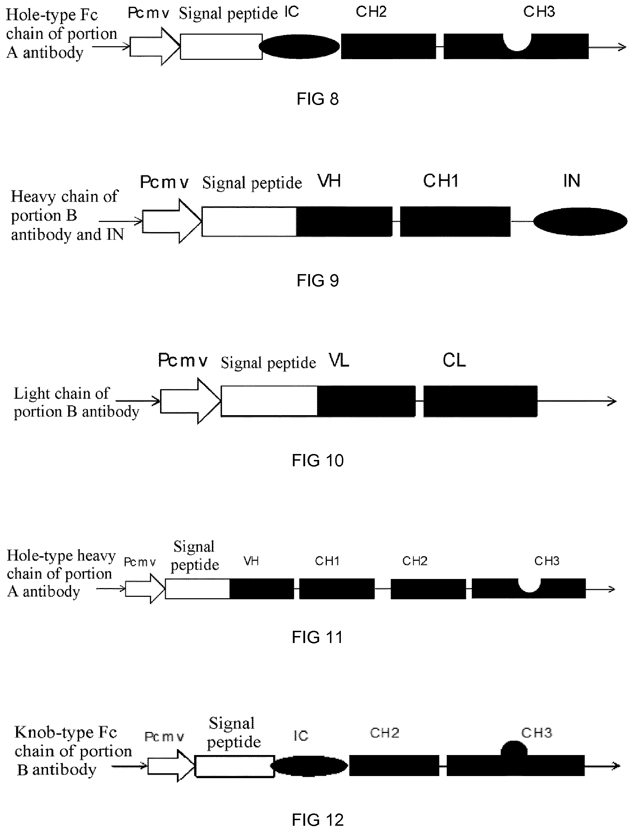

1.3.a. As shown (in FIG. 6), the sequence of the light chain of the portion A antibody is a light chain sequence of natural antibody A. As shown (in FIG. 7), in the CH3 region of the heavy chain of the portion A antibody, T (threonine) at position 366 is mutated to W (tryptophan) to form a "Knobs" structure; meanwhile S (serine) at position 354 is mutated to C (cysteine). As shown (in FIG. 8), in the CH3 region of the IC+Fc (Fc having C-fragment of the split intein fused to the N terminal) region of the portion A antibody, T (threonine) at position 366 is mutated to S (serine), L (leucine) at position 368 is mutated to A (alanine), and Y (tyrosine) at position 407 is mutated to V (valine) to form a "Holes" structure; meanwhile, Y (tyrosine) at position 349 is mutated to C (cysteine). The heavy chain VH+CH1+IN (the heavy chain variable region of the antibody + CH1 region having N-fragment of the split intein fused to the C terminal) of the portion B antibody is as shown (in FIG. 9). As shown (in FIG. 10), the light chain of the portion B antibody is a light chain sequence of natural antibody B.

1.3.b. As shown (in FIG. 6), the sequence of the light chain of the portion A antibody is a light chain sequence of natural antibody A. As shown (in FIG. 11), in the CH3 region of the heavy chain of the portion A antibody, T (threonine) at position 366 is mutated to S (serine), L (leucine) at position 368 is mutated to A (alanine), and Y (tyrosine) at position 407 is mutated to V (valine), to form a "Holes" structure; meanwhile Y (tyrosine) at position 349 is mutated to C (cysteine). As shown (in FIG. 12), in the CH3 region of the IC+Fc (Fc having C-fragment of the split intein fused to the N terminal) region of the portion A antibody, T (threonine) at position 366 is mutated to W (tryptophan) to form a "Knobs" structure; meanwhile, S (serine) at position 354 is mutated to C (cysteine). The heavy chain VH+CH1+IN (the heavy chain variable region of the antibody + CH1 region having N-fragment of the split intein fused to the C terminal) of the portion B antibody is as shown (in FIG. 9). As shown (in FIG. 10), the light chain of the portion B antibody is a light chain sequence of natural antibody B.

1.3.c. Construction of expression vectors of small fragment antibodies as shown in FIG. 5, any one of the antibody fragments is selected from the group C, and N-fragment of the split intein is fused at the position IN shown in FIG. 5. Also, any one of the antibody fragments is selected from the group D, and C-fragment of the split intein is fused at the position IC shown in FIG. 5

1.4. The gene sequences designed in the above 1.3 are subjected to whole-gene chemical synthesis. Restriction endonuclease cleavage sites such as KpnI/BamHI are added at the two sides of the start codon and the stop codon by polymerase chain reaction (PCR). The genes are respectively inserted into a mammalian cell expression vector containing CMV promoter, the subclones are sequenced, and the plasmids are extracted. For transient transfection, a larger amount of plasmid is prepared with a plasmid preparation (omega) from transformed E. coli culture. In addition to the antibody expressing region, the vector includes an origin of replication which allows the plasmid to replicate in E. coli and the β-lactamase gene, which confers ampicillin resistance in E. coli. The transcription unit of an antibody gene consists of a unique restriction site at the 5' end, an immediate early enhancer and promoter from human cytomegalovirus, followed by an intron A sequence, a 5' untranslated region of a human antibody gene, a signal peptide sequence of an immunoglobulin light chain (or other signal peptide sequence), a 3' untranslated region with a signal sequence A, and a unique restriction site at the 3' end, in the case of cDNA construction.

2. For example, standard cell culture techniques described in Current Protocols in Cell Biology (2000), Bonifacino, JS, Dasso, M., Harford, JB, Lippincott-Schwartz, J. and Yamada, KM (ed.), John Wiley & Sons, Inc can be used. The portions A and B antibodies are expressed by transiently co-transfecting HEK293-E cells grown in suspension or HEK29-F cells grown in suspension with various expression vectors, as described below.

2.1. Transient transfection of HEK293-E system: the portions A and B of a bispecific antibody are produced by co-transfecting HEK293-E cells (human embryonic kidney cell line 293 expressing Epstein-Barr virus nuclear antigen; American Type Culture Center, accession number ATCC#CRL-10852, Lot.959 218) respectively with three expression vectors and two expression vectors. The cells are cultured in SFX4HEK293 medium (HyClone) and Gibco Freestyle 293 medium (Gibco) in a ratio of 1:1 to which 100 µg/ml geneticin (Gibco) is added, and the cells are diluted to 1.5-2.5 × 10⁶ cells/ml with fresh medium one day before transfection and incubated at 37°C and 120 rpm in 5% CO₂ for transfection on the following day. Taking a 1 L shaking flask (Coming) as an example, the cells are collected by centrifugation at 500-2000 rpm for 5-10 min on the following day, and then washed several times with (10-50 ml) Gibco Freestyle 293 medium. The cells are collected by centrifugation at 500-2000 rpm for 5-10 min, and then resuspended in 150 ml Gibco Freestyle 293 medium to a cell density of 2-6 × 10⁶ cells/ml in a new 1 L shaking flask (Coming). Plasmids for co-transfection are used in an amount of 0.25-1.5 µg DNA per 10⁶ cells at equimolar ratio of the vectors of genes encoding various chains, and the DNAs are diluted with Gibco Freestyle 293 medium to (40 ng/µL). DNA: PEI (polyscience cationic transfection reagent) =1:2-1:6 are added to the uniformly mixed DNAs and incubated for 5-20 min at room temperature. The cell suspension is added, mixed, and transfected for 4 hours at 37°C and 120 rpm, in 5% CO₂. Equal volume of pre-warmed SFX4HEK293 medium is added after 4 hours, and then 100 µg/ml geneticin (Gibco) is added and incubated at 37°C and 120 rpm, in 5% CO₂ for 5-10 days. The supernatant is directly collected for purification or the supernatant is collected and stored at -80°C.

2.1.a. PEI-mediated co-transfection of HEK293-E cells with three expression vectors of portion A antibody SFX4HEK293 medium (HyClone) and Gibco Freestyle 293 medium (Gibco) are added in a ratio of 1:1, 100 µg/ml geneticin (Gibco) is added, and the cells are diluted to 1.5-2.5 × 10⁶ cells/ml with fresh medium one day before transfection and incubated at 37°C and 120 rpm in 5% CO₂ for transfection on the following day. Taking a 1 L shaking flask (Coming) as an example, the cells are collected by centrifugation at 500-2000 rpm for 5-10 min on the following day, and then washed several times with (10-50 ml) Gibco Freestyle 293 medium. The cells are collected by centrifugation at 500-2000 rpm for 5-10 min, and then resuspended in 150 ml Gibco Freestyle 293 medium to a cell density of 2-6 × 10⁶ cells/ml in a new 1 L shaking flask (Coming). The three expression vectors containing genes encoding the portion A antibody are mixed uniformly at an equimolar ratio in an amount of 0.25-1.5 µg DNA per 10⁶ cells, and the DNAs are diluted with Gibco Freestyle 293 medium to (40 ng/µL). DNA: PEI (polyscience cationic transfection reagent) =1:2-1:6 are added to the uniformly mixed DNAs and incubated for 5-20 min at room temperature. The cell suspension is added, mixed, and transfected for 4 hours at 37°C and 120 rpm, in 5% CO₂. Equal volume of pre-warmed SFX4HEK293 medium is added after 4 hours, and then 100 µg/ml geneticin (Gibco) is added and incubated at 37°C and 120 rpm, in 5% CO₂ for 5-10 days, to obtain the portion A antibody. The supernatant is directly collected for purification or the supernatant is collected and stored at -80°C.

2.1.b. PEI-mediated co-transfection of HEK293-E cells with two expression vectors of portion B antibody SFX4HEK293 medium (HyClone) and Gibco Freestyle 293 medium (Gibco) are added in a ratio of 1:1, 100 µg/ml geneticin (Gibco) is added, and the cells are diluted to 1.5-2.5 × 10⁶ cells/ml with fresh medium one day before transfection and incubated at 37°C and 120 rpm in 5% CO₂ for transfection on the following day. Taking a 1 L shaking flask (Coming) as an example, the cells are collected by centrifugation at 1000 rpm for 5 min on the following day, and then washed several times with 50 ml Gibco Freestyle 293 medium. The cells are collected by centrifugation at 1000 rpm for 5 min, and then resuspended in 150 ml Gibco Freestyle 293 medium to a cell density of 2-6 × 10⁶ cells/ml in a new 1 L shaking flask (Coming). The two expression vectors containing genes encoding the portion A antibody are mixed uniformly at an equimolar ratio in an amount of 0.25-1.5 µg DNA per 10⁶ cells, and the DNAs are diluted with Gibco Freestyle 293 medium to (40 ng/µL). DNA: PEI (polyscience cationic transfection reagent) =1:2-1:6 are added to the uniformly mixed DNAs and incubated for 5-20 min at room temperature. The cell suspension is added, mixed, and transfected for 4 hours at 37°C and 120 rpm, in 5% CO₂. Equal volume of pre-warmed SFX4HEK293 medium is added after 4 hours, and then 100 µg/ml geneticin (Gibco) is added and incubated at 37°C and 120 rpm, in 5% CO₂ for 5-10 days, to obtain the portion B antibody. The supernatant is directly collected for purification or the supernatant is collected and stored at -80°C.

3. Protein L affinity purification of antibody in fermentation liquor: The protein is purified from the filtered cell culture supernatant following a standard procedure. Briefly, the antibody is loaded to protein L affinity chromatography (GE healthcare) and washed with PBS (containing 20 mM phosphate, 150 mM NaCl pH 6.8-7.4). The impurity components are washed off with 100 mM citrate buffer at pH 5.0, and the antibody is eluted with 100 mM citrate buffer at pH 3.0, and then immediately neutralized with 1 M tris-Hcl buffer at pH 9.0. A portion of the sample is provided for subsequent protein analysis by for example, SDS-PAGE. The monomeric antibody components are pooled for subsequent in-vitro trans splicing mediated by the intein. If necessary, the monomeric antibody components are concentrated using the MILLIPORE Amicon Ultra (30 MWCO) centrifugal concentrator, frozen and stored at -20°C or -80°C.

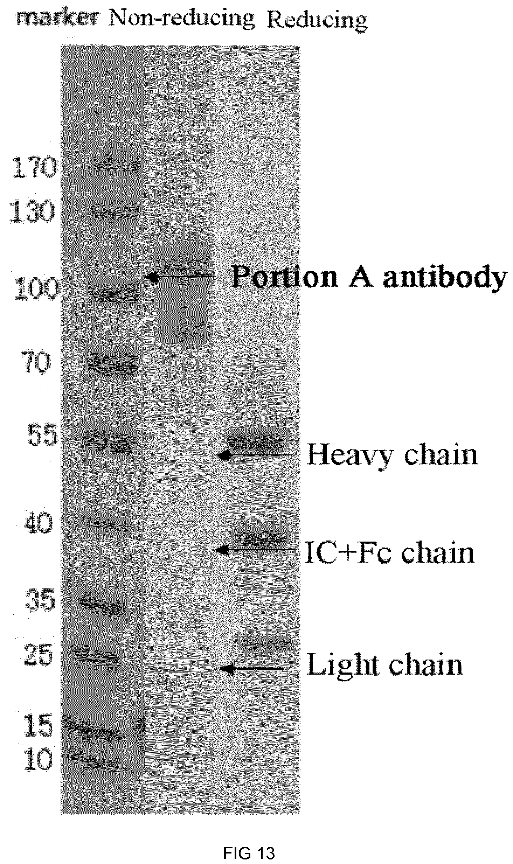

3.1. Protein L affinity purification of portion A antibody in fermentation liquor co-transfected with three expression vectors. The protein is purified from the filtered cell culture supernatant following a standard procedure. The supernatant from which the cells are filtered off is mixed with PBS (containing 20 mM phosphate, and 150 mM NaCl pH 6.8-7.4), flow through a Protein L affinity chromatographic column pre-equilibrated with PBS, and washed with PBS after loading. The impurity components are washed off with 100 mM citrate buffer at pH 5.0, and the antibody is eluted with 100 mM citrate buffer at pH 3.0, and then immediately neutralized with 1 M tris-Hcl buffer at pH 9.0. A portion of the sample is provided for subsequent protein analysis by, for example, SDS-PAGE. As shown in (FIG. 13), in the non-reduced sample, assembled portion A antibody of the bispecific antibody appears at around 103 KD. In the reduced sample, the heavy chain of 55 KD, the IC +Fc chain of 40 KD, and the light chain of 25KD appear. The monomeric antibody components are pooled for subsequent in-vitro trans splicing mediated by the intein. If necessary, the monomeric antibody components are concentrated using the MILLIPORE Amicon Ultra (30 MWCO) ultrafiltration centrifuge tube, frozen and stored at -20°C or -80°C.

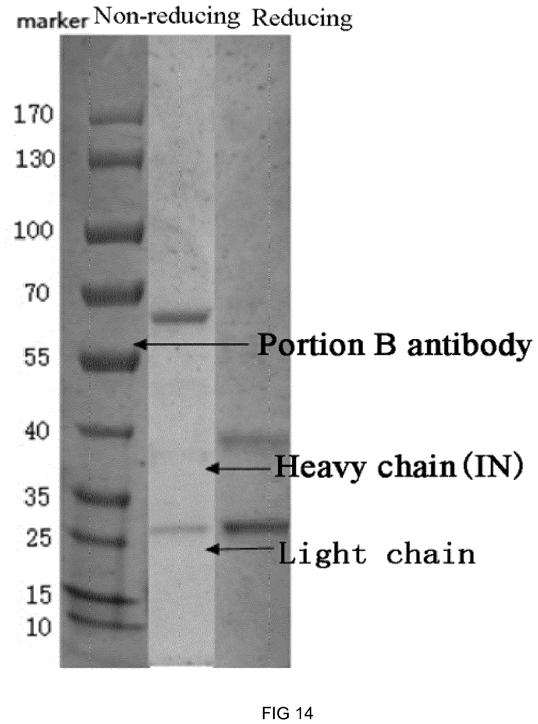

3.2. Protein L affinity purification of portion B antibody in fermentation liquor co-transfected with two expression vectors. The protein is purified from the filtered cell culture supernatant following a standard procedure. The supernatant from which the cells are filtered off is mixed with PBS (containing 20 mM phosphate, and 150 mM NaCl pH 6.8-7.4), run through a Protein L affinity chromatographic column pre-equilibrated with PBS, and washed with PBS after loading. The impurity components are washed off with 100 mM citrate buffer at pH 5.0, and the antibody is eluted with 100 mM citrate buffer at pH 3.0, and then immediately neutralized with 1 M tris-Hcl buffer at pH 9.0. A portion of the sample is provided for subsequent protein analysis by, for example, SDS-PAGE. As shown in (FIG. 14), in the non-reduced sample, assembled portion B antibody of the bispecific antibody appears at around 60 KD. In the reduced sample, the VH+CH1+IN of 35 KD and the light chain of 25 KD appear. The monomeric antibody components are pooled for subsequent in-vitro trans splicing mediated by the intein. If necessary, the monomeric antibody components are concentrated using the MILLIPORE Amicon Ultra (30 MWCO) ultrafiltration centrifuge tube, frozen and stored at -20°C or -80°C.

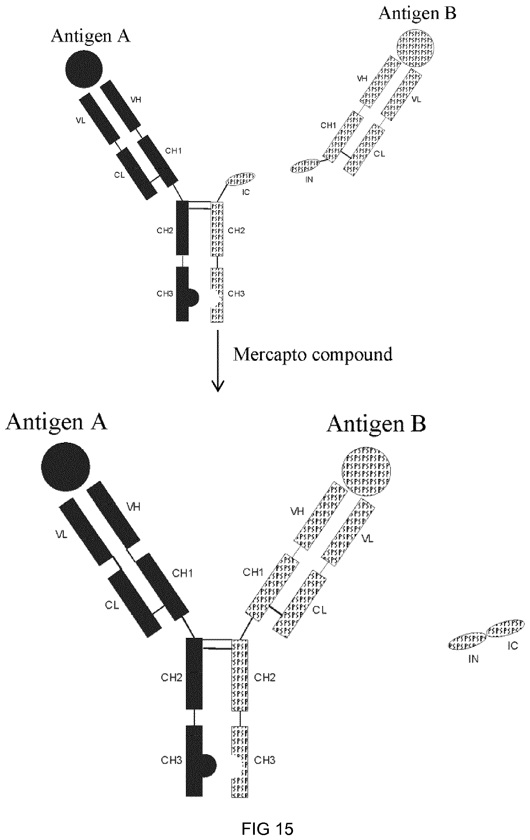

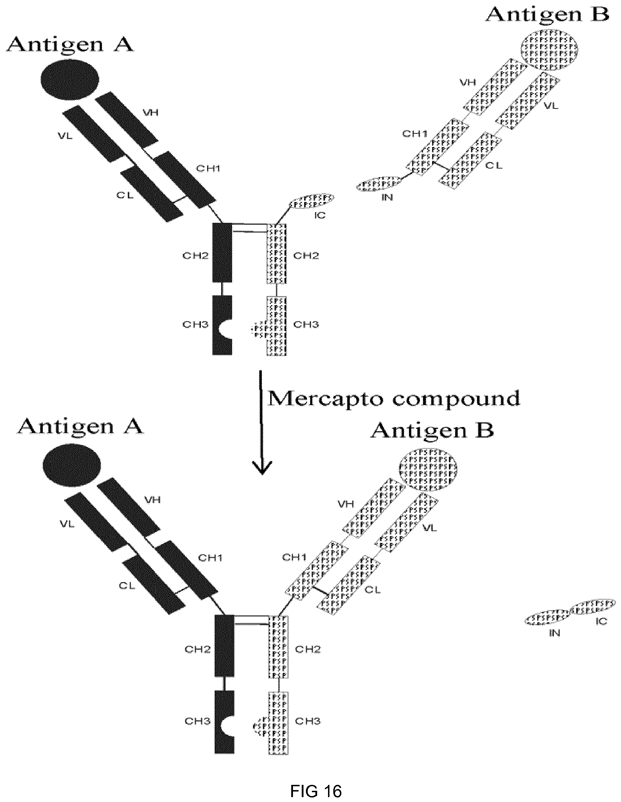

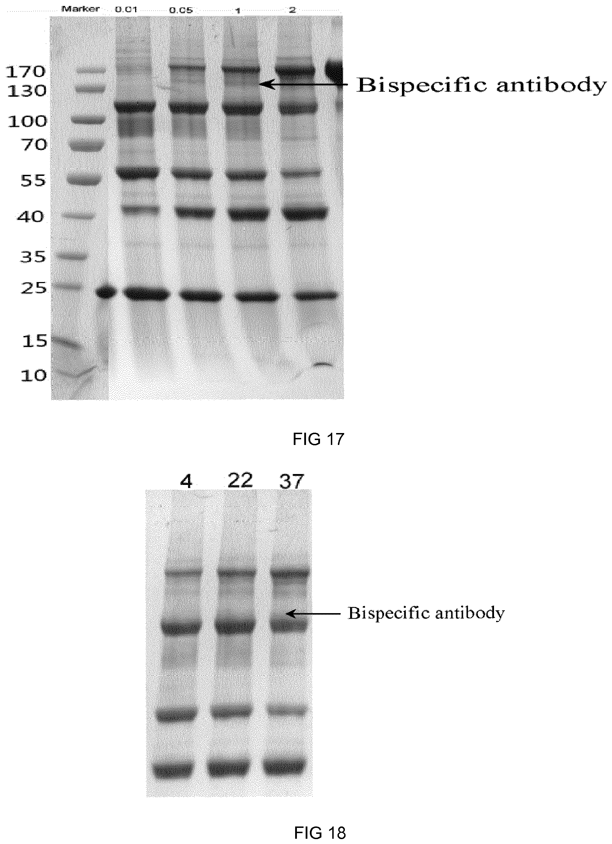

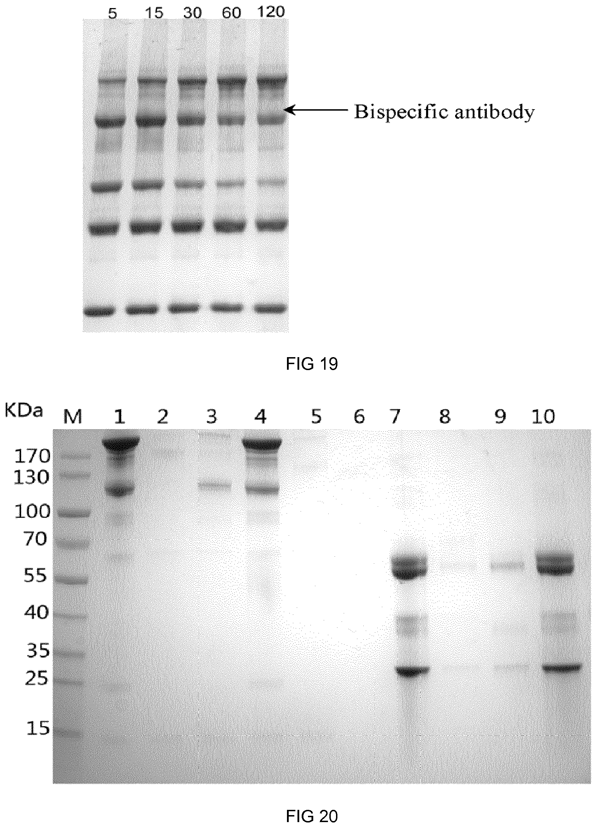

4.1. The in-vitro trans-splicing mediated by the split intein of the portions A and B is as shown in (in Figs. 15 and 16). The portion A and B antibodies purified in Step 3 are mixed at a molar ratio of 1:1, and 0.05 mM to 2 mM DTT or β-mercaptoethanol is added. As shown in (FIG. 17), the final concentration of DTT is 0.01 mM, 0.05 mM, 1 mM, 2 mM respectively. The results show that DTT can induce the occurrence of split intein-mediated trans-splicing at a concentration of 0.05 mM, and an obvious band of the bispecific antibody appears at 150 KD. Trans-splicing mediated by the split intein is induced to occur by a sulfhydryl compound such as TCEP. 1 mM DTT or TCEP is added to the splicing reaction system at 4 - 37°C, and stood respectively at 4, 22, and 37°C. As shown (in FIG. 18), the reaction occurs at 4°C, the reaction efficiency is higher at 22 and 37°C, and an obvious band of the bispecific antibody appears at 150 KD. 1 mM DTT is added to the splicing reaction system, and stood at 37°C for 5 min, 15 min, 30 min, 60 min, and 120 min, respectively. As shown in (FIG. 19), a bispecific antibody is produced at 5 min, and the reaction reaches a plateau at 60 min. At the end of the reaction, the sulfhydryl compound needed to be removed, and the sulfhydryl compound could be removed by adding an oxidizing agent such as hydrogen peroxide, or removed by dialysis. Further, the sulfhydryl compound might be diluted to below a working concentration by high-fold dilution with a buffer to achieve the purpose of terminating the reaction. The reaction is terminated and a sample is taken for detection by non-reducing SDS-PAGE.

4.2. The in-vitro trans-splicing mediated by the split intein of the portions C and D is as described in 4.1.

5.1. Protein A purification of product obtained after trans-splicing mediated by split intein of portions A and B The protein is purified from the reaction mixture of Step 4 following a standard procedure. The sample is mixed with PBS (containing 20 mM phosphate, and 150 mM NaCl pH 6.8-7.4), run through a Protein A affinity chromatographic column pre-equilibrated with PBS, and washed with PBS after loading. The impurity components are washed off with 100 mM citrate buffer at pH 5.0, and the antibody i eluted with 100 mM citrate buffer at pH 3.0, and then immediately neutralized with 1 M tris-Hcl buffer at pH 9.0. A portion of the sample is provided for subsequent protein analysis by, for example, SDS-PAGE. As shown in (FIG. 20), in the non-reduced sample, an obvious band of a bispecific antibody formed by trans-splicing mediated by the split intein appears at 150 KD and the purity is high. In the reduced sample, only a heavy chain of about 50 KD and a light chain of about 25 KD appear. The monomeric antibody component is pooled. If necessary, the monomeric antibody component is concentrated using the MILLIPORE Amicon Ultra (30 MWCO) ultrafiltration centrifuge tube, frozen and stored at -20°C or - 80°C; or purified by, for example, ion exchange chromatography, hydrophobic chromatography, and molecular exclusion chromatography, to achieve a higher purity.

5.2. Purification of product formed by trans-splicing of portions C and D mediated by split intein for the product obtained by trans-splicing of the portions C and D, purification by recombinant protein purification methods such as ion exchange chromatography, hydrophobic chromatography and size exclusion chromatography is required.

Specific applications are shown in the following examples, which are illustrative but do not limit the scope of the claims.

### Example 1: Construction of CD3xHer2 bispecific antibody

### 1.1. Construction of expression vectors

For the construction of expression vectors, general information about the nucleotide sequences of light and heavy chains of human immunoglobulin is provided in Kabat, EA, et al., Sequences of Proteins of Immunological Interest, 5th Edition, Public Health Services, National Institutes of Health, Bethesda, MD. (1991) and in the drugbank database. The amino acids in the antibody chain are numbered and referenced according to the EU numbering (Edelman, G.M., et al., Proc.Natl.Acad.Sci.USA 63(1969)78-85; Kabat, E.A., et al., Sequences of Proteins of Immunological Interest, 5th Edition, Public Health Service, National Institutes of Health, Bethesda, MD. (1991)). The CD3 antibody sequence was derived from humanized OKT3 antibody sequence and the desired gene segments were prepared by oligonucleotides prepared through chemical synthesis. The 600-1800 bp long gene segment was assembled by annealing and ligation of PCR-amplified oligonucleotides, and then cloned into an expression vector via the indicated restriction sites such as KpnI/BamHI. The DNA sequence of the subcloned gene fragment was verified by DNA sequencing. Infomax's VectorNTI Advance suite version 8.0 was used for sequence construction, mapping, analysis, annotation, and description. In order to solve the problem of mispairing of heavy chains, "Knobs-into-Holes" was introduced, the VH and CH1 regions of one heavy chain are removed, and IC (C-fragment of the split intein) was fused to the N-hinge region of CH2. Thus, the heavy-chain homodimer component formed by the heavy chain that cannot be purified and removed was completely prevented. In order to introduce the "Knobs-into-Holes" structure, (threonine) at position 366 in a CH3 region of the CD3 antibody was mutated to W (tryptophan) to form a "Knobs" structure. T (threonine) at position 366 in a CH3 region of the Her2 antibody was mutated to S (serine), L (leucine) at position 368 was mutated to A (alanine), and Y (tyrosine) at position 407 was mutated to V (valine), to form a "Holes" structure. In addition, in order to enhance the binding stability of the CH3 regions, S (serine) at position 354 of the "Knob" chain was mutated to C (cysteine), and Y (tyrosine) at position 349 on the "Hole" chain was mutated to C (cysteine) to enhance the stability between heavy chains by introducing a pair of inter-heavy chain disulfide bonds.

1.1.a. The CD3 antibody was used as the portion A antibody, and the expression vector of each chain was respectively designed on the basis of the following. A light chain of the portion A antibody was designed as shown (in FIG. 6), a knob heavy chain of the portion A antibody was designed as shown (in FIG. 7), and a hole Fc chain of the portion A antibody was designed as shown (in FIG. 8). The Her2 antibody was used as the portion B antibody, and the expression vector of each chain was respectively designed on the basis of the following. A heavy chain IN of the portion B antibody was designed as shown (in FIG. 9) and a light chain of the portion B antibody was designed as shown (in FIG. 10).

1.1.b. The CD3 antibody was used as the portion A antibody, and the expression vector of each chain was respectively designed on the basis of the following. A light chain of the portion A antibody was designed as shown (in FIG. 6), a hole heavy chain of the portion A antibody was designed as shown (in FIG. 11), and a knob Fc chain of the portion A antibody was designed as shown (in FIG. 12). The Her2 antibody was used as the portion B antibody, and the expression vector of each chain was respectively designed on the basis of the following. A heavy chain IN of the portion B antibody was designed as shown (in FIG. 9) and a light chain of the portion B antibody was designed as shown (in FIG. 10).

1.1.c. The Her2 antibody was used as the portion A antibody, and the expression vector of each chain was respectively designed on the basis of the following. A light chain of the portion A antibody was designed as shown (in FIG. 6), a knob heavy chain of the portion A antibody was designed as shown (in FIG. 7), and a hole Fc chain of the portion A antibody was designed as shown (in FIG. 8). The CD3 antibody was used as the portion B antibody, and the expression vector of each chain was respectively designed on the basis of the following. A heavy chain IN of the portion B antibody was designed as shown (in FIG. 9) and a light chain of the portion B antibody was designed as shown (in FIG. 10).

1.1.d. The Her2 antibody was used as the portion A antibody, and the expression vector of each chain was respectively designed on the basis of the following. A light chain of the portion A antibody was designed as shown (in FIG. 6), a hole heavy chain of the portion A antibody was designed as shown (in FIG. 11), and a knob Fc chain of the portion A antibody was designed as shown (in FIG. 12). The CD3 antibody was used as the portion B antibody, and the expression vector of each chain was respectively designed on the basis of the following. A heavy chain IN of the portion B antibody was designed as shown (in FIG. 9) and a light chain of the portion B antibody was designed as shown (in FIG. 10).

### 1.2. Expression of transiently transfected HEK-293E cells

Transient transfection of HEK293-E system. The portions A and B of a bispecific antibody were produced by co-transfecting HEK293-E cells (human embryonic kidney cell line 293 expressing Epstein-Barr virus nuclear antigen; American Type Culture Center, accession number ATCC#CRL-10852, Lot.959 218) respectively with three expression vectors and two expression vectors. The cells were cultured in SFX4HEK293 medium (HyClone) and Gibco Freestyle 293 medium (Gibco) in a ratio of 1:1 to which 100 µg/ml geneticin (Gibco) was added, and the cells were diluted to 1.5-2.5 × 10⁶ cells/ml with fresh medium one day before transfection and incubated at 37°C and 120 rpm in 5% CO₂ for transfection on the following day. Taking a 1 L shaking flask (Coming) as an example, the cells were collected by centrifugation at 1000 rpm for 5 min on the following day, and then washed once with (50 ml) Gibco Freestyle 293 medium. The cells were collected by centrifugation at 1000 rpm for 5 min, and then resuspended in 150 ml Gibco Freestyle 293 medium to a cell density of 4 × 10⁶ cells/ml in a new 1 L shaking flask (Coming). Plasmids for co-transfection were used in an amount of 0.5 µg DNA per 10⁶ cells at equimolar ratio of the vectors of genes encoding various chains, and the DNAs were diluted with Gibco Freestyle 293 medium to (40 ng/µL). DNA: PEI (polyscience cationic transfection reagent) =1:3 were added to the uniformly mixed DNAs and incubated for 20 min at room temperature. The cell suspension was added, mixed, and transfected for 4 hours at 37°C and 110 rpm, in 5% CO₂. Equal volume of pre-warmed SFX4HEK293 medium was added after 4 hours, and then 100 µg/ml geneticin (Gibco) was added and incubated at 37°C and 130 rpm, in 5% CO₂ for 10 days. The supernatant was directly collected for purification or the supernatant was collected and stored at -80°C.

1.2.a. PEI-mediated co-transfection of HEK293-E cells with three expression vectors of portion A antibody constructed in 1.1.a. The cells were cultured in SFX4HEK293 medium (HyClone) and Gibco Freestyle 293 medium (Gibco) in a ratio of 1:1 to which 100 µg/ml geneticin (Gibco) was added, and the cells were diluted to 1.5-2.5 × 10⁶ cells/ml with fresh medium one day before transfection and incubated at 37°C and 120 rpm in 5% CO₂ for transfection on the following day. Taking a 1 L shaking flask (Coming) as an example, the cells were collected by centrifugation at 1000 rpm for 5 min on the following day, and then washed once with (50 ml) Gibco Freestyle 293 medium. The cells were collected by centrifugation at 1000 rpm for 5 min, and then resuspended in 150 ml Gibco Freestyle 293 medium to a cell density of 4 × 10⁶ cells/ml in a new 1 L shaking flask (Coming). Plasmids for co-transfection were used in an amount of 0.5 µg DNA per 10⁶ cells at equimolar ratio of the vectors of genes encoding various chains, and the DNAs were diluted with Gibco Freestyle 293 medium to (40 ng/µL). DNA: PEI (polyscience cationic transfection reagent) =1:3 were added to the uniformly mixed DNAs and incubated for 20 min at room temperature. The cell suspension was added, mixed, and transfected for 4 hours at 37°C and 110 rpm, in 5% CO₂. Equal volume of pre-warmed SFX4HEK293 medium was added after 4 hours, and then 100 µg/ml geneticin (Gibco) was added and incubated at 37°C and 130 rpm, in 5% CO₂ for 10 days. The supernatant was directly collected for purification or the supernatant was collected and stored at -80°C.

1.2.b. PEI-mediated co-transfection of HEK293-E cells with three expression vectors of portion A antibody constructed in 1.1.b. The cells were cultured in SFX4HEK293 medium (HyClone) and Gibco Freestyle 293 medium (Gibco) in a ratio of 1:1 to which 100 µg/ml geneticin (Gibco) was added, and the cells were diluted to 1.5-2.5 × 10⁶ cells/ml with fresh medium one day before transfection and incubated at 37°C and 120 rpm in 5% CO₂ for transfection on the following day. Taking a 1 L shaking flask (Coming) as an example, the cells were collected by centrifugation at 1000 rpm for 5 min on the following day, and then washed once with (50 ml) Gibco Freestyle 293 medium. The cells were collected by centrifugation at 1000 rpm for 5 min, and then resuspended in 150 ml Gibco Freestyle 293 medium to a cell density of 4 × 10⁶ cells/ml in a new 1 L shaking flask (Coming). Plasmids for co-transfection were used in an amount of 0.5 µg DNA per 10⁶ cells at equimolar ratio of the vectors of genes encoding various chains, and the DNAs were diluted with Gibco Freestyle 293 medium to (40 ng/µL). DNA: PEI (polyscience cationic transfection reagent) =1:3 were added to the uniformly mixed DNAs and incubated for 20 min at room temperature. The cell suspension was added, mixed, and transfected for 4 hours at 37°C and 110 rpm, in 5% CO₂. Equal volume of pre-warmed SFX4HEK293 medium was added after 4 hours, and then 100 µg/ml geneticin (Gibco) was added and incubated at 37°C and 130 rpm, in 5% CO₂ for 10 days. The supernatant was directly collected for purification or the supernatant was collected and stored at -80°C.

1.2.c. PEI-mediated co-transfection of HEK293-E cells with two expression vectors of portion B antibody constructed in 1.1.c. The cells were cultured in SFX4HEK293 medium (HyClone) and Gibco Freestyle 293 medium (Gibco) in a ratio of 1:1 to which 100 µg/ml geneticin (Gibco) was added, and the cells were diluted to 1.5-2.5 × 10⁶ cells/ml with fresh medium one day before transfection and incubated at 37°C and 120 rpm in 5% CO₂ for transfection on the following day. Taking a 1 L shaking flask (Coming) as an example, the cells were collected by centrifugation at 1000 rpm for 5 min on the following day, and then washed once with (50 ml) Gibco Freestyle 293 medium. The cells were collected by centrifugation at 1000 rpm for 5 min, and then resuspended in 150 ml Gibco Freestyle 293 medium to a cell density of 4 × 10⁶ cells/ml in a new 1 L shaking flask (Coming). Plasmids for co-transfection were used in an amount of 0.5 µg DNA per 10⁶ cells at equimolar ratio of the vectors of genes encoding various chains, and the DNAs were diluted with Gibco Freestyle 293 medium to (40 ng/µL). DNA: PEI (polyscience cationic transfection reagent) =1:3 were added to the uniformly mixed DNAs and incubated for 20 min at room temperature. The cell suspension was added, mixed, and transfected for 4 hours at 37°C and 110 rpm, in 5% CO₂. Equal volume of pre-warmed SFX4HEK293 medium was added after 4 hours, and then 100 µg/ml geneticin (Gibco) was added and incubated at 37°C and 130 rpm, in 5% CO₂ for 10 days. The supernatant was directly collected for purification or the supernatant was collected and stored at -80°C.

1.2.d. PEI-mediated co-transfection of HEK293-E cells with two expression vectors of portion B antibody constructed in 1.1.d. The cells were cultured in SFX4HEK293 medium (HyClone) and Gibco Freestyle 293 medium (Gibco) in a ratio of 1:1 to which 100 µg/ml geneticin (Gibco) was added, and the cells were diluted to 1.5-2.5 × 10⁶ cells/ml with fresh medium one day before transfection and incubated at 37°C and 120 rpm in 5% CO₂ for transfection on the following day. Taking a 1 L shaking flask (Coming) as an example, the cells were collected by centrifugation at 1000 rpm for 5 min on the following day, and then washed once with (50 ml) Gibco Freestyle 293 medium. The cells were collected by centrifugation at 1000 rpm for 5 min, and then resuspended in 150 ml Gibco Freestyle 293 medium to a cell density of 4 × 10⁶ cells/ml in a new 1 L shaking flask (Coming). Plasmids for co-transfection were used in an amount of 0.5 µg DNA per 10⁶ cells at equimolar ratio of the vectors of genes encoding various chains, and the DNAs were diluted with Gibco Freestyle 293 medium to (40 ng/µL). DNA: PEI (polyscience cationic transfection reagent) =1:3 were added to the uniformly mixed DNAs and incubated for 20 min at room temperature. The cell suspension was added, mixed, and transfected for 4 hours at 37°C and 110 rpm, in 5% CO₂. Equal volume of pre-warmed SFX4HEK293 medium was added after 4 hours, and then 100 µg/ml geneticin (Gibco) was added and incubated at 37°C and 130 rpm, in 5% CO₂ for 10 days. The supernatant was directly collected for purification or the supernatant was collected and stored at -80°C.

### 1.3. Protein L affinity purification of antibody in fermentation liquor

The protein was purified from the filtered cell culture supernatant following a standard procedure. Briefly, the antibody was subjected to protein L affinity chromatography (GE healthcare) and washed with PBS (containing 20 mM phosphate, 150 mM NaCl pH 6.8-7.4). The impurity components were washed off with 100 mM citrate buffer at pH 5.0, and the antibody was eluted with 100 mM citrate buffer at pH 3.0, and then immediately neutralized with 1 M tris-Hcl buffer at pH 9.0. A portion of the sample was provided for subsequent protein analysis by for example, SDS-PAGE. The monomeric antibody components were pooled for subsequent in-vitro trans splicing mediated by the intein. If necessary, the monomeric antibody components were concentrated using the MILLIPORE Amicon Ultra (30 MWCO) centrifugal concentrator, frozen and stored at -20°C or -80°C.

1.3.a. Protein L affinity purification of antibody in cell fermentation liquor obtained in Step 1.2.a. Protein L affinity purification of portion A antibody in fermentation liquor co-transfected with three expression vectors - The protein was purified from the filtered cell culture supernatant following a standard procedure. The supernatant from which the cells were filtered off was mixed with PBS (containing 20 mM phosphate, and 150 mM NaCl pH 6.8-7.4), run through a Protein L affinity chromatographic column pre-equilibrated with PBS, and washed with PBS after loading. The impurity components were washed off with 100 mM citrate buffer at pH 5.0, and the antibody was eluted with 100 mM citrate buffer at pH 3.0, and then immediately neutralized with 1 M tris-Hcl buffer at pH 9.0. A portion of the sample was provided for subsequent protein analysis by, for example, SDS-PAGE. As shown in (FIG. 13), in the non-reduced sample, assembled portion A antibody of the bispecific antibody appears at around 103 KD. In the reducing electrophoresis, the heavy chain of 55 KD, the IN +Fc chain of 40 KD, and the light chain of 25KD appear. The monomeric antibody component was pooled, which might be purified to obtain a purified product comprising mainly portion A antibody for subsequent in-vitro trans-splicing mediated by the intein. If necessary, the monomeric antibody components were concentrated using the MILLIPORE Amicon Ultra (30 MWCO) ultrafiltration centrifuge tube, frozen and stored at -20°C or - 80°C.

1.3.b. Protein L affinity purification of antibody in cell fermentation liquor obtained in Step 1.2.b. Protein L affinity purification of portion A antibody in fermentation liquor co-transfected with three expression vectors - The protein was purified from the filtered cell culture supernatant following a standard procedure. The supernatant from which the cells were filtered off was mixed with PBS (containing 20 mM phosphate, and 150 mM NaCl pH 6.8-7.4), run through a Protein L affinity chromatographic column pre-equilibrated with PBS, and washed with PBS after loading. The impurity components were washed off with 100 mM citrate buffer at pH 5.0, and the antibody was eluted with 100 mM citrate buffer at pH 3.0, and then immediately neutralized with 1 M tris-Hcl buffer at pH 9.0. A portion of the sample was provided for subsequent protein analysis by, for example, SDS-PAGE. As shown in (FIG. 13), in the non-reduced sample, assembled portion A antibody of the bispecific antibody appears at around 103 KD. In the reducing electrophoresis, the heavy chain of 55 KD, the IN +Fc chain of 40 KD, and the light chain of 25KD appear. The monomeric antibody component was pooled, which might be purified to obtain a purified product comprising mainly portion A antibody for subsequent in-vitro trans-splicing mediated by the intein. If necessary, the monomeric antibody components were concentrated using the MILLIPORE Amicon Ultra (30 MWCO) ultrafiltration centrifuge tube, frozen and stored at -20°C or - 80°C.

1.3.c. Protein L affinity purification of antibody in cell fermentation liquor obtained in Step 1.2.c. The protein was purified from the filtered cell culture supernatant following a standard procedure. The supernatant from which the cells were filtered off was mixed with PBS (containing 20 mM phosphate, and 150 mM NaCl pH 6.8-7.4), run through a Protein L affinity chromatographic column pre-equilibrated with PBS, and washed with PBS after loading. The impurity components were washed off with 100 mM citrate buffer at pH 5.0, and the antibody was eluted with 100 mM citrate buffer at pH 3.0, and then immediately neutralized with 1 M tris-Hcl buffer at pH 9.0. A portion of the sample was provided for subsequent protein analysis by, for example, SDS-PAGE. As shown in (FIG. 14), in the non-reduced sample, assembled portion B antibody of the bispecific antibody appears at around 60 KD. In the reduced sample, the VH+CH1+IN of 35 KD and the light chain of 25 KD appear. The monomeric antibody component was pooled, which might be purified to obtain a purified product comprising mainly portion B antibody for subsequent in-vitro trans-splicing mediated by the intein. If necessary, the monomeric antibody components were concentrated using the MILLIPORE Amicon Ultra (30 MWCO) ultrafiltration centrifuge tube, frozen and stored at -20°C or -80°C.

1.3.d. Protein L affinity purification of antibody in cell fermentation liquor obtained in Step 1.2.d. The protein was purified from the filtered cell culture supernatant following a standard procedure. The supernatant from which the cells were filtered off was mixed with PBS (containing 20 mM phosphate, and 150 mM NaCl pH 6.8-7.4), run through a Protein L affinity chromatographic column pre-equilibrated with PBS, and washed with PBS after loading. The impurity components were washed off with 100 mM citrate buffer at pH 5.0, and the antibody was eluted with 100 mM citrate buffer at pH 3.0, and then immediately neutralized with 1 M tris-Hcl buffer at pH 9.0. A portion of the sample was provided for subsequent protein analysis by, for example, SDS-PAGE. As shown in (FIG. 14), in the non-reduced sample, assembled portion B antibody of the bispecific antibody appears at around 60 KD. In the reduced sample, the VH+CH1+IN of 35 KD and the light chain of 25 KD appear. The monomeric antibody component was pooled, which might be purified to obtain a purified product comprising mainly portion B antibody for subsequent in-vitro trans-splicing mediated by the intein. If necessary, the monomeric antibody components were concentrated using the MILLIPORE Amicon Ultra (30 MWCO) ultrafiltration centrifuge tube, frozen and stored at -20°C or -80°C.

### 1.4. In-vitro trans-splicing mediated by the split intein of the portions A and B

As shown (in Figs. 15 and 16), the portion A and B antibodies purified in Step 1.3 were mixed at a molar ratio of 1:1, and 0.05 mM to 2 mM DTT or β-mercaptoethanol was added. As shown in (FIG. 17), the final concentration of DTT is 0.01 mM, 0.05 mM, 1 mM, and 2 mM respectively. The results show that DTT can induce the occurrence of split intein-mediated trans-splicing at a concentration of 0.05 mM, and an obvious band of the bispecific antibody appears at 150 KD. Trans-splicing mediated by the split intein was induced to occur by a sulfhydryl compound such as TCEP. 1 mM DTT or TCEP was added to the splicing reaction system at 4 - 37°C, and stood respectively at 4, 22, and 37°C. As shown (in FIG. 18), the reaction occurs at 4°C, the reaction efficiency is higher at 22 and 37°C, and an obvious band of the bispecific antibody appears at 150 KD. 1 mM DTT was added to the splicing reaction system, and stood at 37°C for 5 min, 15 min, 30 min, 60 min, and 120 min, respectively. As shown in (FIG. 19), a bispecific antibody is produced at 5 min, and the reaction reaches a plateau at 60 min. At the end of the reaction, the sulfhydryl compound needed to be removed, and the sulfhydryl compound could be removed by adding an oxidizing agent such as hydrogen peroxide, or removed by dialysis. Further, the sulfhydryl compound might be diluted to below a working concentration by high-fold dilution with a buffer to achieve the purpose of terminating the reaction. The reaction was terminated and a sample was taken for detection by non-reducing SDS-PAGE.

### 1.5. Protein A purification of product obtained after trans-splicing mediated by split intein of portions A and B

The protein was purified from the reaction mixture of Step 4 following a standard procedure. The sample was mixed with PBS (containing 20 mM phosphate, and 150 mM NaCl pH 6.8-7.4), run through a Protein A affinity chromatographic column pre-equilibrated with PBS, and washed with PBS after loading. The impurity components were washed off with 100 mM citrate buffer at pH 5.0, and the antibody was eluted with 100 mM citrate buffer at pH 3.0, and then immediately neutralized with 1 M tris-Hcl buffer at pH 9.0. A portion of the sample was provided for subsequent protein analysis by, for example, SDS-PAGE, as shown in (FIG. 20). FIG. 20 shows Coomassie blue staining of the product eluate from rProteinA chromatography in SDS-PAGE, in which M. marker; 1. before loading (N); 2. eluate from Ni column (N); 3. eluate 1 from rProteinA chromatography (N); 4. eluate 2 from rProteinA chromatography (N); 5. eluate 3 from rProteinA chromatography; 6. empty; 7. before loading (R) ;8. eluate from Ni column (R); 9. eluate 1 from ProteinA chromatography (R) 10. eluate 2 from rProteinA chromatography (R), where N denote Nonreducing, and R denote Reducing. It can be known from FIG. 20, in the non-reduced sample, an obvious band of a bispecific antibody formed by trans-splicing mediated by the split intein appears at 150 KD and the purity is high. In the reduced sample, only a heavy chain of about 50 KD and a light chain of about 25 KD appear. The monomeric antibody component was pooled. If necessary, the monomeric antibody component was concentrated using the MILLIPORE Amicon Ultra (30 MWCO) ultrafiltration centrifuge tube, frozen and stored at -20°C or - 80°C; or purified by, for example, ion exchange chromatography, hydrophobic chromatography, and molecular exclusion chromatography, to achieve a higher purity. FIG. 21 is a schematic view showing products prepared through methods for preparing a multi-specific antibody and an immune hybrid protein, in which A shows a product group A prepared through a method for preparing a multi-specific antibody and an immune hybrid protein (bispecific antibodies, antibody-directed immunotoxins, and other products); B shows a product group B prepared through a method for preparing a multi-specific antibody and an immune hybrid protein (hybrid proteins of antibody fragments with cytokines or toxins); and C shows a product group C prepared through a method for preparing a multi-specific antibody and an immune hybrid protein (bi- to tetra-specific antibodies, immune hybrid proteins, and other products).

In summary, in the present disclosure, in order to solve the problem of mispairing of heavy chains, "Knobs-into-Holes" is introduced, the VH and CH1 regions of one heavy chain are removed, and IC (C-fragment of the split intein) is fused to the N-hinge region of CH2. Thus, the heavy-chain homodimer component formed by the heavy chain that cannot be purified and removed is completely prevented. In order to introduce the "Knobs-into-Holes" structure, (threonine) at position 366 in a CH3 region is mutated to W (tryptophan) to form a "Knob" structure. T (threonine) at position 366 in a CH3 region of another heavy chain is mutated to S (serine), L (leucine) at position 368 is mutated to A (alanine), and Y (tyrosine) at position 407 is mutated to V (valine), to form a "Hole" structure. In addition, in order to enhance the binding stability of the CH3 regions, S (serine) at position 354 of the "Knob" chain is mutated to C (cysteine), and Y (tyrosine) at position 349 on the "Hole" chain is mutated to C (cysteine) to enhance the stability between heavy chains by introducing a pair of inter-heavy chain disulfide bonds. Also, more importantly, an intact "Knob" heavy chain and "Hole" Fc chain are co-expressed. Due to the high difference in the properties of the "Knob" heavy chain homodimer and the "Hole" Fc homodimer from the target product, a heterodimer of the "Knob" heavy chain and the "Hole" Fc, separation and purification can be carried out simply. Therefore, the problem of mispairing of heavy chains can be completely avoided in the final product.

In the present disclosure, a bispecific antibody is split into an antigen A binding portion and an antigen B binding portion for the first time, as shown (in Figs. 2 and 3), which are expressed separately, and then ligated into an intact antibody by protein trans-splicing by a split intein. The two light chains do not exist at the same time, and the two VH+CH1 chains do not exist at the same time, so there is no case where the light chain of A binds to the heavy chain of B, either the case where the light chain of B binds to A. Therefore, the situation of mispairing of light chains is avoided completely.

In the present disclosure, the trans-splicing function of the split intein is combined with the construction of bispecific antibodies for the first time, and portion A and B antibodies expressed and purified separately are linked to form an intact antibody by means of the am-splicing function of the split intein. This kind of specific antibodies is similar in structure to naturally occurring antibody molecules, thereby avoiding the instability of antibody molecules due to structural differences and the high immunogenicity in vivo.

In the present disclosure, recombinant gene expression technology is used to produce a bispecific antibody, and the sequence used may be a humanized antibody sequence or a fully human antibody sequence, to finally obtain a humanized or fully human bispecific antibody. This will greatly reduce the immunogenicity of the bispecific antibody in vivo, laying a foundation for the use of the bispecific as a drug.

Since the portion A antibody retains the entire Fc region, the bispecific antibody obtained by trans-splicing mediated by the intein also retains the entire Fc region, thus retaining the effector functions of the antibody, such as complement-dependent cytotoxicity (CDC) or antibody dependent cellular cytotoxicity (ADCC) and extended half-life of binding to FcRn (Fc receptor) in vascular endothelium.

In the present disclosure, both portion A and B antibodies are expressed in a mammalian cell expression system, for example, by transiently transfecting 293E, 293F, or CHO cells, and stably transfecting CHO cells. The products expressed by mammalian cells are glycosylated and more similar in structure to natural antibody molecules. The bispecific antibodies obtained by intein-mediated trans-splicing are well glycosylated and have well maintained stability of the bispecific antibody molecules, and antibody effects such as ADCC, CDC, etc., the in vivo half-life is prolonged, and the duration of the effect of action of the drug is increased.

In the method for preparing a bispecific antibody according to the present disclosure, the purification process is easy and convenient in operation. First, both portions A and B can be recovered by chromatography with a high recovery rate, such as ProteinL or ProteinA/G affinity chromatography. The bispecific antibody obtained by intein-mediated trans-splicing can be recovered by chromatography with a high recovery rate, such as ProteinA/G affinity chromatography, which can facilitate the subsequent hydrophobic chromatography, or ion exchange chromatography, and other operations. Therefore, the difficulty of purification is greatly reduced and a high-quality product can be obtained.

Since the hinge region between CH1 and CH2 of the antibody heavy chain is flexible and the primary Fab region in the Fc region of the antibody is substantially identical in structure, the method is applicable to the production of any bispecific antibodies with no need to perform property analysis based on the nature of each antibody. The present disclosure is fully applicable to the production of bispecific antibodies of any of the antibody subtypes (IgG, IgA, IgM, IgD, IgE, IgM, and light chain kappa and lambda), thus having broad versatility.

### Example 2: Preparation of immunotoxin Herceptin-PE38KDEL by taking advantage of the trans-splicing function of Npu DnaE

### 2.1. Construction of Her HC expressing vector pCEP4-Her HC-Nn having N-fragment of split intein Npu DnaE fused

Using the primers shown in Table 1, and using the synthesized heavy-chain nucleic acid molecule of Herceptin comprising a coding signal peptide gene as a template, the gene encoding the heavy chain of Herceptin was cloned. Using the synthesized nucleic acid molecule comprising Npu DnaE as a template, the gene encoding the N-fragment of Npu DnaE was cloned. The multiplication was carried out using the PrimerStar Max available from TaKaRa, under PCR conditions including 30 cycles of 10 s at 94°C, 10 s at 55°C, and 10 s at 72°C. The obtained fragments were recovered by agarose gel electrophoresis, and the gene encoding the heavy chain of Herceptin and the gene encoding the N-fragment of Npu DnaE were sequentially synthesized by overlap PCR, in which the N-fragment of Npu DnaE was at the C terminal of the fusion peptide. PCR conditions included 30 cycles of 10 s at 94°C, 10 s at 55°C, and 10 s at 72°C. The gene fragment was treated with *Hin*dIII and *Bam*HI*,* and ligated to pCEP4 that was also treated with *Hind*III and *Bam*HI*.* The plasmid structure was as shown in the figure. The ligated product was transformed into E. coli DH5α competent cells, and the transformed cells were plated on an agar plate containing 50 µg/mL ampicillin overnight. The monoclonal clones grown on the plate were picked and cultured in 5 mL of LB medium containing 50 µg/mL ampicillin with agitation overnight, and the plasmid was extracted and sequenced. The sequencing results indicated that the constructed Her HC-Nn sequence was correct.

**Table 1**

| Primer name | Primer sequence | Enzymatic cleavage site |
|---|---|---|
| Sig-Her-For (SEQ ID NO.1) | 5’- AAAAAGCTTATGGGTTGGAGCTGCATCATCT TGTTCCTCG-3’ | *Hind*III |
| Her-HC-OL-Nn-Rev | 5'-GCCACCACCTGATCCGCCTCCGCCTGA CTTCCCGGGGCTCAGG-3' | |
| (SEQ ID NO.2) | | |
| Nn-OL-HerHC-For | 5’-GGCGGATCTGGTGGTGGCGGATCT TATTGTTTAAGCTATGAAACGGAAATATTG- 3’ | |
| (SEQ ID NO.3) | | |
| Nn-Rev-BamHI | 5'-AAA GGATCC TCA GTGGTGGTGGTGGTGGT GGTGGTGGTGGTGATTCGGCAAATTATCAAC CCG-3' | *Bam*HI |
| (SEQ ID NO.4) | | |

### 2.2. Expression and purification of fusion polypeptide Herceptin-Nn

The fusion peptide Herceptin-Nn was expressed using a transient expression system in the HEK293-E system. HEK293-E cells (human embryonic kidney cell line 293 expressing Epstein-Barr virus nuclear antigen; American Type Culture Center, accession number ATCC#CRL-10852, Lot.959 218) were cultured in SFX4HEK293 medium (HyClone) and Gibco Freestyle 293 medium (Gibco) in a ratio of 1:1 to which 100 µg/ml geneticin (Gibco) was added. The cells were diluted to 1.5-2.5 × 106 cells/ml with fresh medium one day before transfection and incubated at 37°C and 120 rpm in 5% CO₂ for transfection on the following day. On the day of transfection, pCEP4-Her HC-Nn was mixed with the constructed Herceptin light chain expression vector pCEP4-Her LC at an equal weight ratio in an amount of 0.5 µg DNA per 10⁶ cells. The DNAs were diluted with Gibco Freestyle 293 medium to (40 ng/µL), and DNA: PEI = 1:3 were added to the uniformly mixed DNAs and incubated for 20 min at room temperature for later use. The cells were collected by centrifugation at 1000 rpm for 5 min, washed once with Gibco Freestyle 293 medium, collected by centrifugation at 1000 rpm for 5 min, and then resuspended at a cell density of 4 × 10⁶ cells/ml in 150 ml Gibco Freestyle 293 medium in a new 1 L shaking flask (Coming). The incubated DNA-PEI complex was added to the cells, and transfected at 37°C and 110 rpm in 5% CO₂ for 4 hours. Subsequently, an equal volume of pre-warmed SFX4 HEK293 medium, and 100 µg/ml geneticin (Gibco) were added, and the incubation was continued for 10 days at 37°C and 130 rpm, in 5% CO₂. The supernatant was directly collected for purification or the supernatant was collected and stored at -80°C.

The collected supernatant was 1:1 mixed with PBS (20 mM PBS, 150 mM NaCl, pH 6.8-7.4), and loaded onto a Protein A affinity chromatographic column equilibrated with PBS in advance. After loading, the column was washed with 10 times volume of PBS, the antibody was eluted with 100 mM citrate buffer (pH 3.0), and the collected eluted sample was immediately neutralized with 1 M Tris-HCl buffer (pH 9.0). A small sample was taken for SDS-PAGG analysis. The non-reduced sample showed assembled Herceptin-Nn at around 170 kD, and a Her HC-Nn chain of about 70 kD and a light chain of 25 KD appeared in the reduced sample. Samples containing the protein of interest were pooled for subsequent split intein-mediated in-vitro splicing If necessary, the monomeric antibody components were concentrated using the MILLIPORE Amicon Ultra (30 MWCO) ultrafiltration centrifuge tube, frozen and stored at -20°C or -80°C.

### 3. Preparation of Herceptin-PE38KDEL by taking advantage of the trans-splicing function of Npu DnaE

The obtained fusion polypeptide Nc-PE38KDEL and the fusion polypeptide Herceptin-Nn were mixed at a molar ratio of 1:1, and DTT was added at a final concentration of 1 mM, and incubated at 25°C for 60 min. Samples were taken for analysis by SDS-PAGE and Western Blot.

### 4. Separation and purification of Herceptin-PE38KDEL produced by Npu DnaE-mediated trans-splicing

Herceptin-PE3 8KDEL was purified by using Ni²⁺ NTA to capture the fusion polypeptide in Step 3 that was not amenable to trans-splicing reaction and the by-product produced by trans-splicing. A Ni²⁺ gravity column was packed, the volume of the column was 1 ml, washed with 5 column volumes of water, and then equilibrated with 10 column volumes of a binding buffer (20 mM PBS, 500 mM NaCl, 20 mM imidazole, pH 7.5). The reaction system obtained in Step 3 was loaded onto the column. The flow rate was 1 ml/min, and the flow-through was collected. After loading, the column was washed with 5 column volumes of an elution buffer containing 40 mM imidazole (20 mM PBS, 500 mM NaCl, 40 mM imidazole, pH 7.5), and the washing was collected, followed by washing with 10 column volumes of an elution buffer containing 150 mM imidazole (20 mM PBS, 500 mM NaCl, 150 mM imidazole, pH 7.5). The protein samples collected for SDS-PAGE were, if necessary, frozen at -20°C or stored at -80°C, or purified to achieve a higher purity, by for example ion exchange chromatography, hydrophobic chromatography, and size exclusion chromatography.

There are many shortcomings in the traditional methods for producing immunotoxins. For example, chemical reagents are required in the chemical coupling method, and the product is not uniform due to the large number of modification sites in the targeting polypeptide portion, and the chemical coupling bond is prone to breakage, leading to the leakage of toxicity. In the strategy of directly expressing the immunotoxin fusion protein, if a prokaryotic expression system is used, the target protein is often in the form of an inclusion body, the refolding efficiency is low and the steps are complicated. If an eukaryotic expression system is used, the expression of an immunotoxin may be limited by its natural toxicity to host cells. The method of the present disclosure achieves many advantages: 1. In the method of the present disclosure, only different targeting polypeptide portion and toxic polypeptide portion are needed to be prepared, and then combined to produce an immunotoxin which can target a different target and possess a different toxicity mechanism, with significant diversity and flexibility. 2. In the method of the present disclosure, the targeting polypeptide portion and the toxic polypeptide portion can be separately expressed in a suitable host cell. Where a special folding environment, especially advanced post-translational modifications, is/are needed, the expression can be carried out in mammalian cells. Where the polypeptide has no too much modification requirements, the expression can take place in *E. coli.* By means of the separate expression of the polypeptide of interest in a suitable expression system, higher production, yield and purity can be achieved. 3. In the method of the present disclosure, the linkage between the targeting polypeptide portion and the toxic polypeptide portion is site-specific, such that no by-products are produced, and the resulting product is highly homogeneous. 4. In the method of the present disclosure, the targeting polypeptide portion and the toxic polypeptide portion are linked via a peptide bond by means of the trans-splicing effect of intein, so good stability is exhibited compared with other connection models such as chemical coupling. 5. In the method of the present disclosure, the trans-splicing reaction conditions are mild, the reaction is efficient and can be easily integrated with other process and scaled up, and no harmful substance are needed to be added during the reaction.

## Claims

1. A method for expressing and preparing an antibody fusion protein, wherein said antibody fusion protein comprises:
a light chain and a heavy chain of a first antibody that specifically binds to a first antigen; optionally wherein the heavy chain further comprises an intein-N moiety (IN) linked to its C-terminus;
a light chain and the VH+CH1 portion of a heavy chain of a second antibody that specifically binds to a second antigen, wherein the C-terminus of the CH1 domain is fused to an IN moiety;
an Fc chain from said second antibody, wherein the N-terminus of the Fc chain is linked to an Intein-C moiety (IC); optionally, wherein said Fc chain further comprises an IN moiety linked to its C-terminus;
a knob and a corresponding hole capable of associating with each other, the knob and hole being located in the interface of the CH3 domain of the heavy chain of the first antibody and of the CH3 domain of the Fc chain or vice versa;
wherein the method comprises the following steps:
A1: obtaining and amplifying the coding sequences of a "portion A antibody", said portion A antibody comprises said light chain and said heavy chain of said first antibody, and said Fc chain of said second antibody, in which the Intein-C moiety (IC) is fused to the N-terminus of said Fc chain;
A2: constructing an eukaryotic or prokaryotic expression vector by whole-gene synthesis, wherein the vector comprises the coding sequences amplified in step A1 and obtaining the portion A antibody by expressing the vector via transient transfection or stable transfection; and
B1: obtaining and amplifying the coding sequence of a "portion B antibody", said portion B antibody comprises said light chain and said VH+CH1 chain of said second antibody, in which the C-terminus of said VH+CH1 chain is fused to the Intein-N moiety (IN);
B2: constructing an eukaryotic or prokaryotic expression vector by whole-gene synthesis, wherein the vector comprises the coding sequences amplified in step B1 and obtaining the portion B antibody by expressing the vector via transient transfection or stable transfection; and
D: obtaining the antibody fusion protein by subjecting the portion A antibody, the portion B antibody to protein trans-splicing *in vitro.*

2. The method for expressing and preparing the antibody fusion protein according to claim 1, wherein the method further comprises the following steps:
C1: obtaining and amplifying the coding sequence of a "portion C antibody", said portion C antibody comprises a single chain third antibody, in which IC is fused the N-terminus of said third antibody; and the IC is for binding to the further C-terminal IN of the heavy chain of said first antibody; or, the IC is for binding to the further C-terminal IN of the Fc chain of said second antibody;
and/or,
C2: obtaining and amplifying the coding sequence of a "portion D antibody", said portion D antibody comprises a single chain fourth antibody, in which IC is fused to the N-terminus of said fourth antibody; and the IC is for binding to the further C-terminal IN of the heavy chain of said first antibody; or, the IC is for binding to the further C-terminal IN of the Fc chain of said second antibody; and
D: obtaining the antibody fusion protein by subjecting the portion A antibody, the portion B antibody, the portion C antibody, and/or the portion D antibody to protein trans-splicing in vitro.

3. The method for expressing and preparing the antibody fusion protein according to any one of claims 1-2, wherein in Steps A2 and B2, the expression is performed in a mammalian cell expression system.

4. The method for expressing and preparing the antibody fusion protein according to claim 3, wherein in Steps A2 and B2, the mammalian cell is 293E, 293F or CHO cells.

5. The method for expressing and preparing the antibody fusion protein according to any one of claims 1-4, wherein the product expressed in Steps A2 and B2 is obtained through purification by ProteinL affinity chromatography or by ProteinA/G chromatography; and the fusion protein in Step D is obtained through purification by ProteinA/G chromatography.

6. The method for expressing and preparing the antibody fusion protein according to any one of claims 1-5, wherein in Step D, the in-vitro trans-splicing is mediated by a split intein in the presence of a sulfhydryl compound.

## Patentansprüche

1. Verfahren zur Expression und Herstellung eines Antikörper-Fusionsproteins, wobei das Antikörper-Fusionsprotein umfasst:
eine leichte Kette und eine schwere Kette eines ersten Antikörpers, der spezifisch an ein erstes Antigen bindet; optional wobei die schwere Kette ein Intein-N-Element (IN) umfasst, das an ihren C-Terminus gekoppelt ist;
eine leichte Kette und den VH+CH1-Teil einer schweren Kette eines zweiten Antikörpers, der spezifisch an ein zweites Antigen bindet, wobei der C-Terminus der CH1-Domäne mit einem IN-Element fusioniert ist;
eine Fc-Kette des zweiten Antikörpers, wobei der N-Terminus der Fc-Kette mit einem Intein-C-Element (IC) gekoppelt ist; optional wobei die Fc-Kette ein IN-Element umfasst, das an ihren C-Terminus gekoppelt ist;
eine Noppe und ein dazu passendes Loch, die miteinander assoziieren können, wobei die Noppe und das Loch in der Schnittstelle der CH3-Domäne der schweren Kette des ersten Antikörpers und der CH3-Domäne der Fc-Kette oder umgekehrt lokalisiert sind;
wobei das Verfahren die folgenden Schritte umfasst:
**A1:** Gewinnung und Amplifikation der kodierenden Sequenzen eines "Teil-A-Antikörpers", wobei der Teil-A-Antikörper die leichte Kette und die schwere Kette des ersten Antikörpers sowie die Fc-Kette des zweiten Antikörpers umfasst, bei der das Intein-C-Element (IC) an den N-Terminus der Fc-Kette fusioniert ist;
**A2:** Konstruktion eines eukaryotischen oder prokaryotischen Expressionsvektors durch Ganzgen-Synthese, wobei der Vektor die in Schritt A1 amplifizierten kodierenden Sequenzen umfasst, und Gewinnung des Teil-A-Antikörpers durch Expression des Vektors mittels transienter oder stabiler Transfektion; und
**B1:** Gewinnung und Amplifikation der kodierenden Sequenz eines "Teil-B-Antikörpers", wobei der Teil-B-Antikörper die leichte Kette und die VH+CH1-Kette des zweiten Antikörpers umfasst, bei der der C-Terminus der VH+CH1-Kette mit dem Intein-N-Element (IN) fusioniert ist;
**B2:** Konstruktion eines eukaryotischen oder prokaryotischen Expressionsvektors durch Ganzgen-Synthese, wobei der Vektor die in Schritt B1 amplifizierten kodierenden Sequenzen enthält, und Gewinnung des Teil-B-Antikörpers durch Expression des Vektors mittels transienter oder stabiler Transfektion; und
**D:** Gewinnung des Antikörper-Fusionsproteins, indem der Teil-A-Antikörper, der Teil-B-Antikörper Protein-Trans-Spleißen in vitro unterzogen werden.

2. Verfahren zur Expression und Herstellung des Antikörper-Fusionsproteins nach Anspruch 1, wobei das Verfahren ferner die folgenden Schritte umfasst:
**C1:** Gewinnung und Amplifikation der kodierenden Sequenz eines "Teil-C-Antikörpers", wobei der Teil-C-Antikörper einen einzelkettigen dritten Antikörper umfasst, bei dem IC an den N-Terminus des dritten Antikörpers fusioniert ist; und das IC zur Bindung an das weitere C-terminale IN der schweren Kette des ersten Antikörpers vorgesehen ist; oder das IC zur Bindung an das weitere C-terminale IN der Fc-Kette des zweiten Antikörpers vorgesehen ist;
und/oder,
**C2:** Gewinnung und Amplifikation der kodierenden Sequenz eines "Teil-D-Antikörpers", wobei der Teil-D-Antikörper einen einzelkettigen vierten Antikörper umfasst, bei dem IC an den N-Terminus des vierten Antikörpers fusioniert ist; und das IC zur Bindung an das weitere C-terminale IN der schweren Kette des ersten Antikörpers vorgesehen ist; oder das IC zur Bindung an das weitere C-terminale IN der Fc-Kette des zweiten Antikörpers vorgesehen ist; und
**D:** Gewinnung des Antikörper-Fusionsproteins, indem der Teil-A-Antikörper, der Teil-B-Antikörper, der Teil-C-Antikörper und/oder der Teil-D-Antikörper Protein-Trans-Spleißen in vitro unterzogen werden.

3. Verfahren zur Expression und Herstellung des Antikörper-Fusionsproteins nach einem der Ansprüche 1-2, wobei in Schritten A2 und B2 die Expression in einem Säugetier-Zell-Expressionssystem durchgeführt wird.

4. Verfahren zur Expression und Herstellung des Antikörper-Fusionsproteins nach Anspruch 3, wobei in Schritten A2 und B2 die Säugetierzelle 293E-, 293F- oder CHO-Zellen ist.

5. Verfahren zur Expression und Herstellung des Antikörper-Fusionsproteins nach einem der Ansprüche 1-4, wobei das in Schritten A2 und B2 exprimierte Produkt durch Aufreinigung mittels Protein-L-Affinitätschromatographie oder mittels Protein-A/G-Chromatographie erhalten wird; und das Fusionsprotein in Schritt D durch Aufreinigung mittels Protein-A/G-Chromatographie erhalten wird.

6. Verfahren zur Expression und Herstellung des Antikörper-Fusionsproteins nach einem der Ansprüche 1-5, wobei in Schritt D das in-vitro-Trans-Spleißen durch ein getrenntes Intein in der Gegenwart einer Sulfhydrylverbindung vermittelt wird.

## Revendications

1. Procédé d'expression et de préparation d'une protéine de fusion d'anticorps, ladite protéine de fusion d'anticorps comprenant:
une chaîne légère et une chaîne lourde d'un premier anticorps qui se lie spécifiquement à un premier antigène; optionnellement, la chaîne lourde comprenant un élément Intein-N (IN) lié à son extrémité C-terminale;
une chaîne légère et la partie VH+CH1 d'une chaîne lourde d'un deuxième anticorps qui se lie spécifiquement à un deuxième antigène, l'extrémité C-terminale du domaine CH1 étant fusionnée à un élément IN;
une chaîne Fc dudit deuxième anticorps, l'extrémité N-terminale de la chaîne Fc étant liée à un élément Intein-C (IC); optionnellement, ladite chaîne Fc comprenant un élément IN lié à son extrémité C-terminale;
un bouton et un trou correspondant capables de s'associer l'un à l'autre, le bouton et le trou étant situés à l'interface du domaine CH3 de la chaîne lourde du premier anticorps et du domaine CH3 de la chaîne Fc ou inversement;
le procédé comprenant les étapes suivantes:
A1: obtention et amplification des séquences codantes d'un « anticorps de partie A », ledit anticorps de partie A comprenant ladite chaîne légère et ladite chaîne lourde dudit premier anticorps ainsi que ladite chaîne Fc dudit deuxième anticorps, dans laquelle l'élément Intein-C (IC) est fusionné à l'extrémité N-terminale de ladite chaîne Fc;
A2: construction d'un vecteur d'expression eucaryote ou procaryote par synthèse de gènes entiers, le vecteur comprenant les séquences codantes amplifiées à l'étape A1 et obtention de l'anticorps de partie A par expression du vecteur via une transfection transitoire ou stable; et
B1: obtention et amplification de la séquence codante d'un « anticorps de partie B », ledit anticorps de partie B comprenant ladite chaîne légère et ladite chaîne VH+CH1 dudit deuxième anticorps, dans laquelle l'extrémité C-terminale de ladite chaîne VH+CH1 est fusionnée à l'élément Intein-N (IN);
B2: construction d'un vecteur d'expression eucaryote ou procaryote par synthèse de gènes entiers, le vecteur comprenant les séquences codantes amplifiées à l'étape B1 et obtention de l'anticorps de partie B par expression du vecteurvia une transfection transitoire ou stable; et
D: obtention de la protéine de fusion d'anticorps en soumettant l'anticorps de partie A, l'anticorps de partie B à trans-épissage de protéines in vitro.

2. Procédé d'expression et de préparation de la protéine de fusion d'anticorps selon la revendication 1, dans lequel le procédé comprend en outre les étapes suivantes:
C1: obtention et amplification de la séquence codante d'un « anticorps de partie C », ledit anticorps de partie C comprenant un troisième anticorps à chaîne unique, dans lequel IC est fusionné à l'extrémité N-terminale dudit troisième anticorps; et l'IC est destiné à se lier à l'élément IN C-terminal supplémentaire de la chaîne lourde dudit premier anticorps ; ou l'IC est destiné à se lier à l'élément IN C-terminal supplémentaire de la chaîne Fc dudit deuxième anticorps;
et/ou,
C2: obtention et amplification de la séquence codante d'un « anticorps de partie D », ledit anticorps de partie D comprenant un quatrième anticorps à chaîne unique, dans lequel IC est fusionné à l'extrémité N-terminale dudit quatrième anticorps; et l'IC est destiné à se lier à l'élément IN C-terminal supplémentaire de la chaîne lourde dudit premier anticorps; ou l'IC est destiné à se lier à l'élément IN C-terminal supplémentaire de la chaîne Fc dudit deuxième anticorps; et
D: obtention de la protéine de fusion d'anticorps en soumettant l'anticorps de partie A, l'anticorps de partie B, l'anticorps de partie C et/ou l'anticorps de partie D à trans-épissage de protéines in vitro.

3. Procédé d'expression et de préparation de la protéine de fusion d'anticorps selon l'une des revendications 1 à 2, dans lequel, aux étapes A2 et B2, l'expression est réalisée dans un système d'expression de cellules de mammifères.

4. Procédé d'expression et de préparation de la protéine de fusion d'anticorps selon la revendication 3, dans lequel, aux étapes A2 et B2, la cellule de mammifère est une cellule 293E, 293F ou CHO.

5. Procédé d'expression et de préparation de la protéine de fusion d'anticorps selon l'une des revendications 1 à 4, dans lequel le produit exprimé aux étapes A2 et B2 est obtenu par purification par chromatographie d'affinité à la protéine L ou par chromatographie à la protéine A/G; et la protéine de fusion à l'étape D est obtenue par purification par chromatographie à la protéine A/G.

6. Procédé d'expression et de préparation de la protéine de fusion d'anticorps selon l'une des revendications 1 à 5, dans lequel, à l'étape D, le trans-épissage in vitro est médié par une intéine scindée en présence d'un composé sulfhydryle.
